# EUROPEAN PATENT SPECIFICATION

(11) **EP 3 795 692 B1**
(45) Date of publication and mention of the grant of the patent: **12.08.2026**
(21) Application number: 18917705.8
(22) Date of filing: 07.05.2018
(51) Int. Cl.: C12Q 1/68, C12Q 1/6883, G16B 20/10, G16B 30/10

(54) **METHOD, APPARATUS, AND SYSTEM FOR DETECTING CHROMOSOME ANEUPLOIDY**
VERFAHREN, VORRICHTUNG UND SYSTEM ZUR DETEKTION VON CHROMOSOMENANEUPLOIDIE
PROCÉDÉ, APPAREIL ET SYSTÈME POUR LA DÉTECTION D'UNE ANEUPLOÏDIE CHROMOSOMIQUE

(43) Date of publication of application: 24.03.2021
(73) Proprietor: GeneMind Biosciences Company Limited, Shenzhen City, Guangdong Province 518023 (CN)
(72) Inventor: ZENG, Lidong, Shenzhen City, Guangdong Province 518023 (CN); WU, Zengding, Shenzhen City, Guangdong Province 518023 (CN); JIN, Huan, Shenzhen City, Guangdong Province 518023 (CN); XU, Weibin, Shenzhen City, Guangdong Province 518023 (CN); LI, Linsen, Shenzhen City, Guangdong Province 518023 (CN); ZHAO, Luyang, Shenzhen City, Guangdong Province 518023 (CN); ZHANG, Meng, Shenzhen City, Guangdong Province 518023 (CN); YAN, Qin, Shenzhen City, Guangdong Province 518023 (CN)
(74) Representative: Novagraaf Group
(86) International application number: PCT/CN2018/085865
(87) International publication number: WO 2019/213810

(56) References cited:
- WO-A1-2014/165596
- WO-A1-2015/184404
- WO-A2-2015/051163
- WO-A2-2017/009372
- CN-A- 107 133 495
- CN-A- 107 403 075
- CN-A- 107 949 845
- R. W. K. CHIU ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, vol. 105, no. 51, 23 December 2008 (2008-12-23), pages 20458 - 20463, XP055284693, ISSN: 0027-8424, DOI: 10.1073/pnas.0810641105

## Description

### TECHNICAL FIELD

The present disclosure relates to the field of bioinformatics, in particular to a method, a device and a system for detecting chromosomal aneuploidy.

### BACKGROUND

Down's syndrome (trisomy 21), Edwards syndrome (trisomy 13) and Patau syndrome (trisomy 18) are the most common neonatal chromosomal aneuploidy diseases, the incidences of which are respectively 1/700 (Papageorgiou, E. A. et al., Fetal-specific DNA methylation ratio permits noninvasive prenatal diagnosis of trisomy 21. Nat. Med. 17, 510-513 (2011)), 1/6,000 and 1/10,000 (Driscoll, D. A. & Gross, S. Prenatal Screening for Aneuploidy. N. Engl. J. Med. 360, 2556-2562 (2009)). These chromosomal aneuploidies can result in very high incidence and mortality. Amniocentesis and chorionic villus sampling are standard methods for diagnosing fetal chromosomal abnormalities, but these diagnosis methods can cause an abortion rate as high as 0.6% to 1.9%. In order to avoid these risks, it is desirable to develop a safer method for Noninvasive Prenatal Testing (NIPT) for aneuploidy abnormalities at a further earlier stage of gestation.

In 1997, Lu Yuming (Lo, Y. M. D. et al., Presence of fetal DNA in maternal plasma and serum. Lancet, 350, 485-487 (1997)) reported for the first time that cell-free fetal DNA (cffDNA) was detected in bodies of pregnant women, which makes it possible to check the genetic conditions of fetuses through maternal blood. It is reported that cffDNA accounts for about 4% to 10% of maternal cell-free DNA in the first trimester and second trimester and reaches 10% to 20% in the third trimester. In 2008, Lu Yuming (Chiu, R. W. K. et al., Noninvasive prenatal diagnosis of fetal chromosomal aneuploidy by massively parallel genomic sequencing of DNA in maternal plasma. Proc. Natl. Acad. Sci. 105, 20458-20463 (2008)) and Setphen Quake (Chitkara, U. et al., Noninvasive diagnosis of fetal aneuploidy by shotgun sequencing DNA from maternal blood. Proc. Natl. Acad. Sci. U. S. A.105, 16266-71 (2008)) each reported the application of Next Generation Sequencing (NGS) in detecting fetal chromosomal aneuploidy abnormalities. At present, more and more sequencing platforms are available for genetic testing.

For the sensitivity and/or accuracy of chromosomal aneuploidy variation detection based on the data of each sequencing platform, there is still room for further improvement. Multiple factors are associated with the sensitivity and/or accuracy of detection. For example, the difference between the lengths of reads generated by different sequencing platforms is great. The length of a read is also referred to as read length which ranges from tens of bp (base pair) to thousands of bp, and the read length at least affects the confidence of reads alignment. For another example, the error rate of sequencing also affects the confidence of reads alignment, and generally, the higher the error rate, the lower the confidence.

International application WO/2014/16559 A1 discloses a method and system for analyzing circulating cell-free nucleic acids from a pregnant female with reduced bias. Counts of sequence reads mapped to portions of a reference genome are obtained. A regression model is generated that models the relationship between the counts and the GC content. The read counts are normalized according to the regression model to remove the GC bias. The normalized counts are used for further analysis, such as the detection of fetal aneuploidy.

International application WO/2017/009372 A2 discloses a method for determining the presence or absence of a fetal chromosomal aneuploidy in a pregnant female, the method comprising the calculation of a parameter p from sequences obtained from a biological sample from said pregnant female. The present teachings equally provide a method for determining the fetal fraction of said sample. International application WO/2015/05116 A2 discloses methods, processes, systems and machines for non-invasive assessment of genetic variations.

Scientific paper "Noninvasive prenatal diagnosis of fetal chromosomal aneuploidy by massively parallel genomic sequencing of DNA in maternal plasma" by Chiu et al., Proc. Natl. Acad. Sci. U.S.A. 105 (51) 20458-20463, https://doi.org/10.1073/pnas.0810641105 (2008) describes a method in which maternal plasma DNA sequences have been quantified for the noninvasive prenatal detection of fetal trisomy 21 using massively parallel genomic sequencing, based on the reasoning that instead of using approaches that target specific gene loci, the use of a locus-independent method would greatly increase the number of target molecules from the aneuploid chromosome that could be analyzed within the same fixed volume of plasma.

Chinese patent application CN 107 403 075 A discloses an alignment method comprising converting each read into a set of short fragments corresponding to the read, and obtaining multiple sets of short fragments; determining corresponding positions of the short fragments in a reference library so as to obtain a first positioning result, wherein the reference library is a Hash table constructed on basis of a reference sequence, the reference library includes a plurality of entries, each entry of the reference library is corresponding to a seed sequence, each seed sequence can be matched with at least one sequence fragment on the reference sequence, and a distance between two seed sequences corresponding to two adjacent entries of the reference library on the reference sequence is less than the length of the short fragments; removing the short fragment positioned to any one of the adjacent entries of the reference library in the first positioning result, and obtaining a second positioning result; and performing extending based on common parts of the short fragments from the same read in the second positioning result so as to obtaining an alignment result of the read.

### SUMMARY

The embodiments of the present disclosure are intended to solve at least one of the technical problems existing in the prior art or at least provide an alternative practical solution.

According to one embodiment of the present disclosure, a method for detecting chromosomal aneuploidy is provided, which comprises: (1) sequencing at least a portion of a nucleic acid in a sample under test to obtain a sequencing result including reads; (2) aligning the reads to a first reference sequence to obtain an alignment result including specific chromosomes to which the reads are mapped, wherein the first reference sequence is a set of regions with an alignment capability of 1 on a reference genome, and the region with an alignment capability of 1 is defined as a region mapped to a unique location on the reference genome and the region has a length that is between 0.5 times and 2 times the length of the reads or the average length of the reads; (3) determining, for a first chromosome, the amount of reads mapped to the first chromosome based on the alignment result; and (4) comparing the amount of the reads mapped to the first chromosome with the amount of reads in a negative control mapped to the first chromosome to determine the number of the first chromosome.

The method comprises using a specific reference sequence to screen and map reads and thus can quickly and simply detect chromosomal aneuploidy to obtain an accurate test result. The method is applicable to detection and analysis of off-line data based on various sequencing platforms, and in particular to detection and analysis of reads containing unknown bases, *i.e.,* processing and analysis of reads containing gaps (usually displayed as N).

According to another embodiment of the present disclosure, a device for detecting chromosomal aneuploidy variation is provided, which is configured for implementing the method for detecting chromosomal aneuploidy in the aforementioned embodiment of the present disclosure. The device comprises: a sequencing module configured for sequencing at least a portion of a nucleic acid in a sample under test to obtain a sequencing result including reads; an alignment module configured for aligning the reads from the sequencing module to a first reference sequence to obtain an alignment result including specific chromosomes to which the reads are mapped, wherein the first reference sequence is a set of regions with an alignment capability of 1 on a reference genome, the region with an alignment capability of 1 is defined as a region mapped to a unique location on the reference genome, and the region has a length that is between 0.5 times and 2 times the length of the reads or the average length of the reads; a quantification module configured for determining, for a first chromosome, the amount of reads mapped to the first chromosome based on the alignment result coming from the alignment module; and a judgment module configured for comparing the amount of the reads mapped to the first chromosome from the quantification module with the amount of reads in a negative control mapped to the first chromosome to determine the number of the first chromosome.

According to another embodiment of the present disclosure, a computer-readable medium is also provided to store/carry a computer-executable program. When the program is executed, all or a part of the steps of the method for detecting chromosomal aneuploidy in the aforementioned embodiment of the present disclosure can be carried out by instruction-related hardware. The medium includes, but is not limited to, read-only memory, random access memory, magnetic disk, optical disk, or the like.

According to yet another embodiment of the present disclosure, provided is a terminal, a system for detecting chromosomal aneuploidy. The system comprises a computer-executable program and a processor which is configured for executing the aforementioned computer-executable program, wherein the execution of the computer-executable program comprises completing the method for detecting chromosomal aneuploidy in the aforementioned embodiment of the present disclosure.

The method, device and/or system for detecting chromosomal aneuploidy provided by any of the aforementioned embodiments can be used to detect chromosomal aneuploidy variation, and detection results obtained have relatively high sensitivity and accuracy. The method is applicable to detection and analysis of off-line data based on various sequencing platforms, and in particular to detection and analysis of reads containing unknown bases, *i.e.,* processing and analysis of reads containing gaps.

The additional aspects and advantages of the embodiments of the present disclosure will be partially set forth in the following description, and will partially become apparent from the following description or be appreciated by practice of the embodiments of the present disclosure.

### BRIEF DESCRIPTION OF THE DRAWINGS

FIG. 1 is a schematic diagram of the distance between two adjacent entries of a reference library in an alignment method employed by a specific embodiment of the present disclosure.
FIG. 2 is a schematic diagram of a connectivity length of the alignment method employed by a specific embodiment of the present disclosure.
FIG. 3 is a schematic diagram of the relation between coefficients of variation and sizes of window in a specific embodiment of the present disclosure.
FIG. 4 is a schematic diagram of the relation between standardized sequencing depths of chromosomes and GC contents of the chromosomes in a specific embodiment of the present disclosure.

### DETAILED DESCRIPTION

The embodiments of the present disclosure are described in detail below, and the examples of the embodiments are shown in the accompanying drawings, throughout which identical or similar reference numerals represent identical or similar elements or elements having identical or similar functions. The embodiments described below by reference to the accompanying drawings are exemplary and are intended to illustrate the disclosure rather than be construed as limiting the disclosure.

In the description of the present disclosure, the terms "first" and "second" are used for description purpose only rather than construed as indicating or implying relative importance or implicitly indicating the number or order of indicated technical features. In the description of the present disclosure, unless otherwise specifically defined, "a plurality of" means two or more.

The sequencing (also referred to as sequence determination) in the description of the present disclosure refers to nucleic acid sequence determination, including DNA sequencing and/or RNA sequencing, and/or including long-read sequencing and/or short-read sequencing.

Sequencing can be performed through a sequencing platform, which may be chosen from, but is not limited to, the Hisq/Miseq/Nextseq sequencing platform (Illumina), the Ion Torrent platform (Thermo Fisher/ Life Technologies), the BGISEQ platform (BGI) and single-molecule sequencing platforms. The sequencing method may be chosen from single-read sequencing and paired-end sequencing. The obtained sequencing results/data (*i.e.,* sequenced piece of nucleic acid) are referred to as reads. The length of a read is referred to as read length.

The embodiments of the present disclosure provide a method for detecting chromosomal aneuploidy including the abnormality of the amount of a chromosome or a part of regions of the chromosome. The method comprises: (1) sequencing at least a portion of a nucleic acid in a sample under test to obtain a sequencing result including reads; (2) aligning the reads to a first reference sequence to obtain an alignment result including specific chromosomes to which the reads are mapped, wherein the first reference sequence is a set of regions with an alignment capability of 1 on a reference genome, and the region with an alignment capability of 1 is defined as a region mapped to a unique location on the reference genome; (3) determining, for a first chromosome, the amount of reads mapped to the first chromosome based on the alignment result; and (4) comparing the amount of the reads mapped to the first chromosome with the amount of reads in a negative control mapped to the first chromosome to determine the number of the first chromosome.

The method comprises using a specific reference sequence to screen and map reads and thus can quickly and simply detect chromosomal aneuploidy to obtain an accurate test result. The method is applicable to detection and analysis of off-line data based on various sequencing platforms, and in particular to detection and analysis of reads containing unknown bases, *i.e.,* processing and analysis of reads containing gaps.

Sequencing can be performed on the entire chromosome (genome), several chromosomes or partial regions of a chromosome. In general, this is mainly related to the characteristics of a target chromosome or region, including the association between the target chromosome or region and other chromosomes or regions.

The alignment used herein refers to sequence alignment, including the process of mapping one or more sequences to another or more sequences and an obtained mapping result. For example, it includes the process of mapping reads to a reference sequence and the process of obtaining a read mapping/matching result as well.

The reference sequence (reference or ref) used herein is the same as a reference chromosome sequence and is a predetermined sequence. It may be a DNA and/or RNA sequence determined and assembled by oneself in advance, a DNA and/or RNA sequence determined and disclosed by others, or any reference template in a biological category to which a sample source individual/target individual obtained in advance belongs, such as all or at least a portion of a disclosed genome assembly sequence of the same biological category. If the sample source individual or target individual is a human, its genome reference sequence (also referred to as reference genome or reference chromosome set) may be chosen from human reference genomes provided by UCSC, NCBI, or ENSEMBL database, such as HG19, HG38, GRCh36, GRCh37, GRCh38, or the like. Those skilled in the art can know the corresponding relation between the aforementioned reference genome versions from the descriptions of the databases to choose a version for use. Further, a resource library containing more reference sequences can also be configured in advance. For example, before alignment, a sequence which is closer to or has certain characteristics is chosen or determined and assembled as a reference sequence according to the gender, race, region and other factors of the target individual, which helps to obtain a more accurate sequence analysis result subsequently. The reference sequence used herein contains the chromosome number and the location information of each site on a chromosome.

The first reference sequence used herein is at least a portion of the reference genome, and it is a version that is constructed by the inventor based on the characteristics of a disclosed off-line data set discovered by mining in combination with the characteristics of the sequencing platform used, off-line data properties (including read length/error rate/data quality and other factors) and an attempt for the purpose of detecting chromosomal aneuploidy. Using the first reference sequence to map reads can help to quickly obtain a mapping result and reduce the amount of data to be processed in the subsequent steps.

In some embodiments, the alignment capability of the regions is determined by the following method: sliding a first window of size of L1 on the reference genome to obtain a plurality of regions; and aligning the region to the reference genome to calculate the alignment capability of the region based on the number of positions in the reference genome to which the region matches. The region or window used herein corresponds to a sequence on the reference genome. The size of the first window and/or the step size of sliding may be set with reference to the purpose of detecting, a variation detecting principle adopted, the read length and the sequence characteristics of the reference genome. Preferably, the step size of sliding is set to be not greater than the size of the first window so as to keep as many regions with an alignment capability of 1 on the reference genome as possible, which can help to increase the utilization rate of off-line data.

In general, L1 may be set according to read length, for example, it can be set as any integer that is 0.5 time to 2 times the read length or average read length; and the step size of sliding may be set as any integer that is less than 0.5 time, 0.2 time or 0.1 time the read length. In one example, the chosen reference genome is HG19 in UCSC database, with read length being 25 bp, L1 being set as 25 bp and the step size of sliding being set as less than 10 bp, 5 bp or 2 bp. For example, the step size of sliding is set as 1 bp, which means the presence of an overlap of (L1-1) bp between two adjacent first windows. Thus, all region sets on the reference genome that meet this specific requirement can be obtained, a sequencing result can be sufficiently utilized to obtain a more comprehensive alignment result, and the utilization rate of data can be increased.

Particularly, in one example, the alignment capability of each region is calculated, and the reciprocal of the number of locations in the reference genome to which the region is aligned is regarded as the alignment capability of the region. For example, if one region is aligned to a unique location of the reference genome, the alignment capability of the region is 1; and if another region can be aligned to five locations of the reference genome, the alignment capability of the region is 1/5.

The first reference sequence may be created when detecting the target sample, or may be created and saved in advance for invoking at detection.

In some embodiments, the first reference sequence is at least a portion of a human reference genome with the regions shown in Table 1 removed. As a large space is needed to illustrate the sequences of all the removed regions, the location/position information of these regions to be removed/blocked in the human reference genome HG19 is shown in Table 1 to represent these regions. It can be understood that these regions may correspond to different start positions on chromosomes in different versions of human reference genomes, but this does not prevent those skilled in the art from determining and blocking the sequences of these regions as shown in the table below to obtain the first reference sequence. The reference sequence with these regions blocked/removed is beneficial for quick implementation of subsequent steps and accurate results.

**Table 1**

| Chrom osome No. | Start position | End position | Chrom osome No. | Start position | End position | Chrom osome No. | Start position | End position | Chrom osome No. | Start position | End position |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 121350000 | 121365000 | 4 | 68265000 | 68280000 | 10 | 42585000 | 42600000 | 22 | 18870000 | 18885000 |
| 1 | 121470000 | 121500000 | 5 | 134250000 | 134265000 | 10 | 127575000 | 127590000 | 23 | 58560000 | 58575000 |
| 1 | 142545000 | 142590000 | 6 | 58770000 | 58785000 | 10 | 135495000 | 135510000 | 24 | 6105000 | 6135000 |
| 1 | 142785000 | 142845000 | 6 | 161025000 | 161040000 | 11 | 51570000 | 51600000 | 24 | 9180000 | 9195000 |
| 1 | 142860000 | 142875000 | 7 | 61785000 | 61800000 | 16 | 33945000 | 33975000 | 24 | 9930000 | 10050000 |
| 1 | 142905000 | 142965000 | 7 | 61965000 | 61980000 | 16 | 46380000 | 46440000 | 24 | 10080000 | 10095000 |
| 1 | 143235000 | 143295000 | 8 | 43080000 | 43110000 | 17 | 22245000 | 22260000 | 24 | 13260000 | 13320000 |
| 1 | 143505000 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 133005000 | 133050000 | 8 | 86730000 | 86745000 | 19 | 8850000 | 8865000 | 24 | 28575000 | 28590000 |
| 2 | 162135000 | 162150000 | 9 | 66960000 | 66975000 | 19 | 27720000 | 27750000 | 24 | 28785000 | 28800000 |
| 2 | 209340000 | 209355000 | 9 | 68400000 | 68700000 | 20 | 29625000 | 29640000 | 24 | 58815000 | 58875000 |
| 3 | 196620000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

In other embodiments, the first reference sequence is at least a portion of the reference genome with regions corresponding to a second window meeting the following condition removed: the sequencing depth of the second window is not less than (greater than or equal to) 4 times the mean sequencing depth of all the second windows, preferably not less than (greater than or equal to) 6 times the mean sequencing depth of all the second windows, *i.e.,* the second windows with sequencing depths far greater than the mean sequencing depth on the reference genome are removed or blocked.

The sequencing depth (also referred to as depth) used herein is the number a region is covered, and can be represented by the ratio of the number of reads mapping to the region to the size of the region. The sequencing depth of the second window is the ratio of the number of reads mapping to the second window to the size of the second window.

The second windows can be acquired by sliding a window of size L2 for the reference genome, giving a series of second windows of size L2. Two adjacent second windows may or may not have an overlap. In one example, the step size of sliding for acquiring the second windows is set as L2, such that no overlap and no interval (zero base overlap and zero base interval) is present between two adjacent second windows. Thus the reference genome is converted into a series of second windows. The series of second windows cover the reference genome once, and can be used to represent the genome.

Specific regions of the reference genome are removed/blocked, such that the influence of some abnormal data on subsequent statistical analysis can be eliminated after alignment in step (2) is performed using the processed reference sequence (the first reference sequence).

According to one specific embodiment of the present disclosure, to second windows with sequencing depths significantly deviating from the mean sequencing depth or the median sequencing depth, a depth can be reassigned to obtain a series of second windows with relatively closer sequencing depths, such that the influence of some abnormal data on subsequent statistical analysis can be eliminated after alignment in step (2) is performed using the first reference sequence containing the series of second windows with the relatively equalized sequencing depths. In one example, the sequencing depth of the second window at the 98th percentile is assigned to the sequencing depths of the second windows over the 98th percentile, or the sequencing depth of the second window at the 99th percentile is assigned to the sequencing depths of the second windows over the 99th percentile, and the first reference sequence acquired in this way can help to eliminate the influence of abnormal data/regions on test results and to obtain accurate test results. For example, all the second windows can be ranked in an ascending order according to the sequencing depth, and a value, for example, the sequencing depth of the second window ranked 99th, is reassigned to the sequencing depths of all the second windows ranked from the 99th to 100th percentiles, so as to eliminate the influence of the windows with abnormal high sequencing depths on subsequent analysis.

The size L2 of the second windows may be adjusted as needed and according to the sequencing results. Preferably, the size of the second windows is expected to be substantially consistent with the size of most of the regions/windows with abnormal high and/or low sequencing depths. In some embodiments, the sample is a human sample, and the reference genome is a human reference genome. Based on preliminary statistics of a sequencing results and/or an alignment result, L2 may be set as 10 Kbp to 20 Kbp, preferably 12 Kbp to 17 Kbp. In one example, the inventor found that when L2 is set as 15 Kbp, more abnormal regions/second windows can be found out.

From the preliminary statistics of the sequencing results, the inventor found that repeated sequence regions generally belong to regions with abnormal sequencing depths. Compared with unprocessed regions, removing such regions/windows with abnormal sequencing depths or reassigning values to such regions can significantly increase the accuracy and/or sensitivity of test results.

Alignment can be performed using known alignment softwares, such as SOAP, BWA, BLAST, MAPQ, TeraMap, or the like, which is not limited by the embodiment. In the process of alignment, according to the setting of alignment parameters, for example, up to n (*e.g.,* set as 1 or 2) mismatched bases may be allowed for a read or a paired reads. If the read has more than n mismatched bases, the read or the paired reads is deemed unable to be aligned to the first reference sequence, or if all the n mismatched bases are in one read in the paired reads , the read in the paired reads is deemed unable to be aligned to the first reference sequence.

According to one specific embodiment of the present disclosure, alignment in step (2) comprises: (a) converting each read into a set of short fragments corresponding to the read to obtain multiple groups of short fragments; (b) determining the corresponding locations of the short fragments in a reference library to obtain a first mapping result, wherein the reference library is a hash table created based on the first reference sequence and contains a plurality of entries, each of which corresponds to a seed sequence matching at least one sequence on the first reference sequence, and the distance between two seed sequences corresponding to two adjacent entries of the reference library on the first reference sequence is less than the length of the short fragment; (c) removing the short fragment mapped to any of the adjacent entries of the reference library from the first mapping result to obtain a second mapping result; (d) extending the sequence based on the short fragments from the same read in the second mapping result to obtain a read alignment result. By the alignment method above, converting the reads into short fragments and converting read sequence information into position/location information (*i.e.,* converting the sequence into digital form), may facilitate quick and accurate alignment of off-line data from various sequencing platforms. This is particularly suitable for the quick and accurate alignment of reads containing unknown bases (*i.e.,* reads containing gaps or Ns), for example, alignment and analysis of reads with poor sequencing quality or base calling, *etc.*

The reference library is substantially a hash table, and can be directly created with seed sequences as keys (key names) and positions/locations of seed sequences on the reference sequence as values (key values); or the reference library is created with numbers or integer strings as keys and positions/locations of the seed sequences on the reference sequence as values after the seed sequences are first converted into the numbers or integer strings. The position/location of the seed sequence on the reference sequence as values may be one or more corresponding positions/locations of the seed sequence on the reference sequence/chromosome, and the positions/locations may be directly represented by a true numerical value or a numerical value range, or may be recorded in customized characters and/or numbers.

In one example, a hash table is constructed using a vector of C++, which may be expressed as follows: *Hash*(*seed*) *= Vector*( *position*). The vector used herein is an object entity that can contain many other elements of the same type, and thus is referred to as a container. The hash table can be saved in the binary system, and thus the reference library is created. In addition, the hash table can also be divided into blocks for storage, with a block head key and a block tail key being set in the block head. For example, for a sequential sequence block {5, 6, 7, 8..., 19, 20}, the block head and the block tail (or table head and table tail) are set as 5 and 20. For a number of 3, as 3 is < 5, it is known that 3 does not belong to the sequential sequence block; for a number of 10, as 10 is > 5 and < 20, it is known that 10 belongs to the sequence block. Thus, when querying, a global index can be selected, or a block can be quickly located by comparing the block head key and the block tail key, without using a global index.

The reference library may be created right before the sequence alignment, or may be created and saved in advance. According to one specific embodiment of the present disclosure, the reference library is created and saved in advance for later use. The creation of the reference library comprises: determining a seed sequence length L according to the total base number (totalBase) of the reference sequence, wherein *L = µ ** log(*totalBase*), *µ* ⊂ [1,3], and L is less than the length of reads (read length) to be aligned and analyzed; generating all possible seed sequences based on the seed sequence length to obtain a seed sequence set; and determining seed sequences capable of mapping to the reference sequence in the seed sequence set and the matching positions of the seed sequences to obtain the reference library. The method is based on the relationship between the seed sequence length and the reference sequence established by the inventor through multiple hypothesis validations, and allows the created reference library to contain complete seed sequences and correlation information of the corresponding position/location of each seed sequence on the reference sequence. The reference library has a compact structure and small memory occupation and can be used for high-speed access and query in sequence mapping analysis. An entry of the reference library obtained according to the embodiment contains only one key, and a key corresponds to at least one value.

The embodiments of the present disclosure do not limit the method for generating all possible seed sequences to obtain a seed sequence set. For an inputted set, the elements in the set can be traversed to obtain a combination of all possible elements with specific lengths, for example, by using a recursive algorithm and/or a loop algorithm.

In one example, for an efficient alignment, the first reference sequence is at least a portion of a human genome containing about 3 billion bases, the length of reads to be processed is not less than 25 bp, and L is an integer selected from [11, 15].

In one example, the first reference sequence is at least a portion of a human cDNA reference genome. The total base number (totalBase) of the reference sequence is counted, a seed sequence (seed) length L is set based on the total base number, wherein *L*(*seed*)=*log*(*totalBase*) * *µ, µ*⊂[1,3]; based on L and bases of DNA sequence including A, T, C and G, a set of all possible seed sequences is generated employing the recursive algorithm to obtain a seed sequence set, which is represented by *seed = B*₁*B*₂...*B_{L}*,*B* ∈ {*ATCG*}; the seed sequences in the seed sequence set capable of matching/aligning to the reference sequence and the matching/aligning positions of the seed sequences are determined, and the reference library is created using the seed sequences capable of matching the reference sequence as keys and the positions of the seed sequences on the reference sequence as values.

In one example, with the first reference sequence as at least a portion of the DNA genome and transcriptome of a species, the total base number (totalBase) of the reference sequence is counted, a seed sequence (seed) length L is set based on the total base number, wherein *L*(*seed*) = *log*(*totalBase*) ** µ, µ*⊂[1,3]; based on L and bases of A, T, C and G for DNA and A, U, C and G for RNA, a set of all possible seed sequences is generated employing the recursive algorithm to obtain a seed sequence set, which is represented by *seed* = *B*₁*B*₂...*B_{L}*,*B* ∈ {*ATCG*}∪{*AUCG*}; the seed sequences in the seed sequence set capable of matching the reference sequence and the matching positions of the seed sequences are determined, and the reference library is created using the seed sequences capable of matching the reference sequence as keys and the positions of the seed sequences on the reference sequence as values.

In one example, each seed sequence can be converted into a string consisting of numeric characters, and a reference library created using the strings as keys may have increased speed for accessing and querying. For example, after a seed sequence capable of matching the first reference sequence is acquired, the seed sequence is coded as follows: ${seed}_{i} = \left\{\begin{matrix}00 & i = A \\ 01 & i = T or U \\ 10 & i = C \\ 11 & i = G\end{matrix}\right. .$ For another example, after a seed sequence set is acquired, a seed sequence in the seed sequence set is coded according to the same base coding scheme as above, and the first reference sequence can also be subjected to a coding process using the same scheme, such that the corresponding position information of the seed sequences on the reference sequence can be acquired quickly and the speed of accessing and querying the created reference library can be increased as well.

According to one specific embodiment of the present disclosure, determining a seed sequence capable of matching/aligning to the first reference sequence in the seed sequence set and the matching/aligning positions of the seed sequence comprises: sliding a window of size L on the first reference sequence, to align the seed sequence in the seed sequence set with window sequence acquired by sliding and to determine seed sequences in the seed sequence set capable of matching the first reference sequence and the matching positions of the seed sequences, wherein the error tolerance of matching is *ε*₁. As such, the corresponding position information of the seed sequence on the first reference sequence can be acquired quickly, which is favorable for the rapid creation of a reference library. The error tolerance is the proportion of allowed mismatched bases, and the mismatch is selected from at least one of substitution, insertion and deletion.

In one example, the matching is a strict matching, *i.e.,* the error tolerance *ε*₁ is zero. When a seed sequence is identical to one or more window sequences, the position of the window sequence is the corresponding position of the seed sequence on the first reference sequence. In another example, the matching is error-tolerant matching, *i.e.,* the error tolerance *ε*₁ is greater than zero. When the proportion of bases in a seed sequence that are not identical to those at the same position of one or more window sequences is less than the error tolerance, the position of the window sequence is the corresponding position of the seed sequence on the first reference sequence. In one example, the corresponding position of seed sequence on the first reference sequence is coded, and a reference library is created using the coding characters (such as numeric characters) as values.

From another perspective, an error tolerance *ε*₁ that is not zero, is equivalent to converting a seed sequence into a set of seed templates permitted by the *ε*₁. For example, if seed = ATCG and the *ε*₁ is 0.25 (that is, one error is allowed in the four bases), the seed template may be ATCG, TTCG, CTCG, GTCG, AACG, ACCG, AGCG, or the like. Determining the position of seed = ATCG on the reference sequence at a *ε*₁ of 0.25 is equivalent to determining the position of all seed templates corresponding to the seed on the first reference sequence. For example, if ref = ATCG, all the seed templates above can match this position; and if ref = TTCG, seed templates ATCG, TTCG, CTCG and GTCG can match this position. The created reference library can take a seed sequence as a key or each of the seed templates corresponding to the seed sequence as a key. Keys are different from one another, and a key corresponds to at least one value. According to one specific embodiment of the present disclosure, when the corresponding position of a seed sequence on the reference sequence is determined, the step size of sliding on the first reference sequence is determined according to L and *ε*₁. In one example, the step size of sliding is not less than *L*ε*₁*.* In one specific example, the first reference sequence is at least a portion of a human genome containing about 3 billion bases, the length of reads to be processed is not less than 25 bp, L is 14 bp, *ε*₁ is 0.2 to 0.3, the step size of sliding is 3 bp to 5 bp, and consequently, two adjacent windows may skip a continuous error combination under the condition of *ε*₁ in the process of sliding and positioning/mapping, which is favorable for rapid positioning/mapping. In one example, the distance between each two adjacent entries of the created reference library is the step size of sliding.

According to one specific embodiment of the present disclosure, (a) comprises: sliding a window of size L for each read to obtain a set of short fragments corresponding to the read, wherein the step size of sliding is 1 bp. As such, for a read of length K, (K-L+1) short fragments of length L are acquired. After the read is converted into short fragments, by quickly accessing and querying the reference library, the corresponding location of each short fragment in the reference library is determined, and thereby the information of the read corresponding to the short fragments can be acquired from the reference library.

According to one specific embodiment of the present disclosure, (b) comprises: aligning the short fragments with seed sequences corresponding to the entries of the reference library to determine the positions of the short fragments in the reference library, wherein the error tolerance of the matching is *ε*₂.

In one example, the matching is a strict matching, *i.e.,* the error tolerance *ε*₂ is zero. When a short fragment is identical to a seed sequence or seed template corresponding to an entry of the reference library, the position/location information of the short fragment in the reference library is acquired.

In another example, the matching is error-tolerant matching, *i.e.,* the error tolerance *ε*₂ is greater than zero. When the proportion of bases of a short fragment unmatched with those of a seed sequence or seed template corresponding to one or more entries of the reference library is less than the error tolerance *ε*₂, the position information of the short fragment in the reference library is acquired.

In one example, *ε*₂= *ε*₁ and is not zero, such that as many effective data as possible can be obtained.

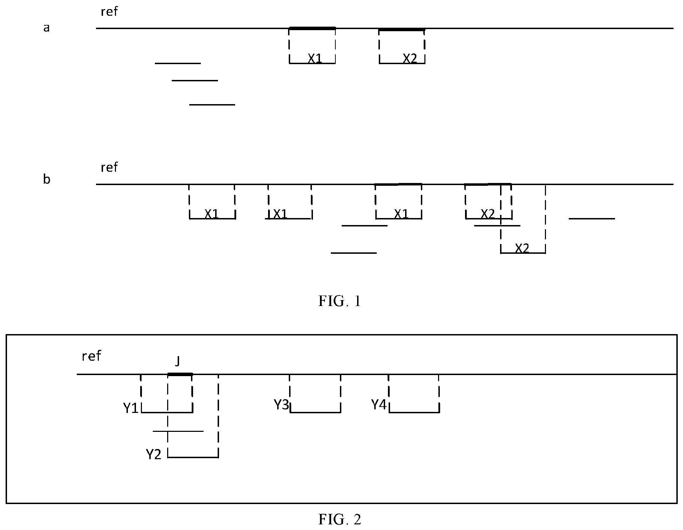

According to one specific embodiment of the present disclosure, referring to FIG. 1, in (b), the distance between two seed sequences X1 and X2 corresponding to two adjacent entries in the reference library on the reference sequence ref may be in the following two cases: when the keys and values of both entries of the reference library are unique, *i.e.,* one entry corresponds to one [key, value], which, referring to FIG. 1a, is equivalent to both X1 and X2 uniquely mapping to the reference sequence (X1 and X2 each maps to only one position on the reference sequence), wherein the distance is the distance between the corresponding positions of X1 and X2 on the reference sequence; when at least one of the two entries of the reference library corresponds to multiple values, which, referring to FIG. 1b, is equivalent to at least one of the two seed sequences X1 and X2 uniquely matches the reference sequence (*i.e.*, at least one of X1 and X2 maps to multiple positions of the reference sequence), wherein the distance is the shortest distance between the positions of X1 and X2 on the reference sequence. This embodiment does not limits the representation of the distance between the two sequences. For example, the distance may be represented by the distance from any of the two termini of one sequence to any of the two termini of the other sequence, or the distance from the center of one sequence to the center of the other sequence.

According to one specific embodiment of the present disclosure, after a second mapping result is acquired, (c) further comprises: removing the short fragments with a connectivity length less than a predetermined threshold to replace the second mapping result with the result after removal, wherein the connectivity length is the overall length of the short fragments from the same read mapped to different entries of the reference library in the second mapping result being mapped to the reference sequence. This processing can help to remove some excessively redundant and/or low-quality data so as to increase the speed of alignment.

The connectivity length may be represented by subtracting the lengths of overlaps of short fragments mapped on the reference sequence from the overall length of the short fragments from the same read mapped to different entries of the reference library. In one example, four short fragments Y1, Y2, Y3 and Y4 from the same read mapped to different entries of the reference library, are of lengths S1, S2, S3 and S4, respectively, wherein the positions/locations of X1 and X2 mapped to the first reference sequence overlap, the length of the overlap is J, and the connectivity length is (S1+S2+S3+S4-J). In one example, the lengths of different short fragments are all L, and the predefined threshold is L. As such, the speed of alignment can be increased under the condition that missing some effective but relatively low-quality data is allowed. According to one specific embodiment of the present disclosure, after the second mapping result is acquired, (c) further comprises: removing a read dissatisfying a predefined requirement by assessing the mapping result of the read according to the mapping result of short fragments from the same read in the second mapping result. While the read is removed, the short fragments corresponding to the read are also removed. As such, under the premise of meeting certain sensitivity and accuracy, accurate matching/rapid local alignment is performed directly based on the second mapping result, which can accelerate the alignment.

This embodiment does not limit the method of assessment, for example, a quantitative scoring method can be employed. In one example, the mapping result of short fragments from the same read is scored. The scoring rule is as follows: a site matching the first reference sequence gives a penalty, and a site unmatched with the first reference sequence gives a bonus; after the second mapping result is acquired, the mapping result of the read is scored according to the mapping result of short fragments from the same read in the second mapping result, and a read with a score not greater than a first preset value is removed.

In one specific example, the read length is 25 bp, and sequences are constructed using short fragments from the same read to give a reconstructed sequence. For example, the base of a site can be determined by support of more short fragments. If a site has no supporting short fragment (*i.e.,* no short fragment match at the site), the base of the site is uncertain and can be represented by N, and thereby a reconstructed sequence can be acquired. It can be seen that the reconstructed sequence corresponds to the read, and the length of the reconstructed sequence is the read length. A site where the reconstructed sequence matches the first reference sequence (ref) gives a one-score penalty, and a site of mismatch with the first reference sequence gives a one-score bonus; the error tolerance of alignment (*i.e.,* the proportion of mismatches allowed for a read/reconstructed sequence) is 0.12, the length of a tolerable error of alignment is 3 bp (25*0.12), the initial score Scoreinit is the read length, and the first preset value is 22 (25-3). As such, a reconstructed sequence with a score less than 22 (*i.e.,* the proportion of sites failing to match the first reference sequence exceeds the error tolerance of alignment) is removed, which can help to accelerate alignment under the condition that missing some effective but relatively low-quality data is allowed.

According to one specific example, bit operation and dynamic programming algorithm (G. Myers. A fast bit-vector algorithm for approximate string matching based on dynamic programming. Journal of the ACM, 46(3): 395-415, 1999) are used; for each reconstructed sequence, the position of each site i is read; rapid match scoring is performed using a 64-bit binary mask, one point for each site; the initial score Scoreinit is the read length, which can be represented by *Scoreᵢₙᵢₜ = length*(*read*); and match scoring is performed to give a score, which can be represented by $Score = \left\{\begin{matrix}- 1 & read \left[i\right] = ref \left[j\right] , j ⊂ \left(i-1,i+1\right) \\ + 1 & read \left[i\right] ! = ref \left[j\right] , j ⊂ \left(i-1,i+1\right)\end{matrix}\right.$.

In one example, the mapping result of short fragments from the same read is scored. The scoring rule is as follows: a position matching the first reference sequence gives a bonus, and a position unmatched with the first reference sequence gives a penalty; after the second mapping result is acquired, the mapping result of the read is scored according to the mapping result of short fragments from the same read in the second mapping result, and short fragments corresponding to the read with a score not less than a second preset value is removed.

In one specific example, the read length is 25 bp, and sequences are constructed using short fragments from the same read to give a reconstructed sequence. For example, the base of a position can be determined by support of more short fragments. If a position has no supporting short fragment (*i.e.,* no short fragment match at the position), the base of the position is uncertain and can be represented by N, and thereby a reconstructed sequence can be acquired. It can be seen that the reconstructed sequence corresponds to the read, and the length of the reconstructed sequence is the read length. A site where the reconstructed sequence matches the first reference sequence (ref) gives a one-score bonus, and a site of mismatch with the first reference sequence gives a one-score penalty; the error tolerance of alignment (*i.e.,* the proportion of mismatches allowed for a read/reconstructed sequence) is 0.12, the length of a tolerable error of alignment is 3 bp (25*0.12), the initial score Scoreinit is -25, and the second preset value is -22 (-25-3). As such, a reconstructed sequence with a score greater than -22 is removed, which can help to accelerate alignment under the condition that missing some effective but relatively low-quality data is allowed.

According to one specific embodiment of the present disclosure, extending the sequence based on the short fragments from the same read in the second mapping result in (d) comprises: constructing a sequence based on short fragments from the same read to give a reconstructed sequence; and extending the sequence based on a common portion of the reconstructed sequence and the reference sequence corresponding to the reconstructed sequence to give an extended sequence. As such, the short fragments and the positioning/mapping information of the short fragments are converted into the positioning information of the read (referred to as a reconstructed sequence herein) corresponding to the short fragments to facilitate a quick and accurate alignment. The common portion is a portion shared by multiple sequences. According to one specific embodiment of the present disclosure, the common portion is a common substring and/or a common subsequence. The common substring refers to a continuous portion shared by multiple sequences, while a common subsequence is not necessarily continuous. For example, for ABCBDAB and BDCABA, the common subsequence is BCBA, and the common substring is AB. In one example of "constructing a sequence based on short fragments from the same read to give a reconstructed sequence", the base of a site on the reconstructed sequence can be determined by having more short fragment supports. If a site has no supporting short fragment (*i.e.*, no short fragment match at the site of the reference sequence), the base of the site is uncertain and can be represented by N, and thereby a reconstructed sequence can be acquired. It can be seen that the reconstructed sequence corresponds to the read, and the length of the reconstructed sequence is the read length.

The reference sequence corresponding to the reconstructed sequence is a reference sequence matching the reconstructed sequence with a length not less than the read length. In one example, the length of the reference sequence corresponding to the reconstructed sequence is the same as that of the reconstructed sequence, both of which are the read length. In another example, error-tolerant matching between the reconstructed sequence and the corresponding reference sequence is allowed. The length of the reference sequence corresponding to the reconstructed sequence is the length of the reconstructed sequence plus double error-tolerant matching length. For example, if the length of the reconstructed sequence (*i.e.,* read length) is 25 bp and the alignment between the reconstructed sequence and the reference sequence allows a mismatching rate of 12%, the reference sequence which the reconstructed sequence matches and 3 bp (25 × 12%) fragments at both termini of the reference sequence can be taken as the reference sequence corresponding to the reconstructed sequence.

According to one specific example of the present disclosure, the common portion is a common substring. Extending the sequence based on the short fragments from the same read in the second mapping result in (d) comprises: searching for a common substring of the reconstructed sequence and the reference sequence corresponding to the reconstructed sequence to determine the longest common substring of the reconstructed sequence and the reference sequence corresponding to the reconstructed sequence; and extending the longest common substring based on an edit distance to give an extended sequence. As such, a more accurate alignment result containing a longer matched sequence can be given.

According to one specific example of the present disclosure, the common portion is a common subsequence. Extending the sequence based on the short fragments from the same read in the second mapping result in (d) comprises: searching for a common subsequence of the reconstructed sequence and the reference sequence corresponding to the reconstructed sequence to determine the longest common subsequence of the reconstructed sequence and the reference sequence corresponding to the reconstructed sequence; and extending the longest common subsequence based on an edit distance to give an extended sequence.

The edit distance, also referred to as Levenshtein distance, means the minimum number of edit operations required by converting one string to the other string. Edit operation includes substituting one character with another, inserting a character, and deleting a character. In general, the shorter the edit distance, the higher the similarity between the two strings.

In one example, for a reconstructed sequence/read, searching for the longest common substring of the reconstructed sequence and the reference sequence corresponding to the reconstructed sequence can be expressed as finding the common substring of two strings *x*₁*x*₂...*xᵢ* and *y*₁*y*₂...*yⱼ,* the lengths of which are respectively m and n. The length *c*[*i, j*] of the common substring of the two strings is calculated to give a transfer equation: $c \left[i, j\right] = \left\{\begin{matrix}0 & i = 0 or j = 0 \\ c \left[i-1,j-1\right] + 1 & {x}_{i} = {y}_{j} \\ 0 & {x}_{i} \neq {y}_{j}\end{matrix}\right. .$ The equation is solved to give the length *max*(*c*[*i,j*]),*i* ∈{1,*...,m*}, *j* ∈ {1,...,*n*} of the longest common substring of the two sequences. Then, by using the edit distance, the longest common substring is converted into a corresponding reference sequence. Both termini of the longest common substring can be extended continuously to find out the minimum number of character operations (substitution, deletion and insertion) required between two strings. The edit distance may be determined by dynamic programming algorithm. The problem has an optimal substructure. The edit distance *d*[*i, j*] can be calculated using the following formula: $d \left[i, j\right] = \left\{\begin{matrix}0 & i = 0 or j = 0 \\ min \left(d\left[i-1,j\right]+gap, d\left[i,j-1\right]+gap,d\left[i-1,j-1\right]+match\right) & {x}_{i} = {y}_{j} \\ min \left(d\left[i-1,j\right]+gap, d\left[i,j-1\right]+gap,d\left[i-1,j-1\right]+mismatch\right) & {x}_{i} \neq {y}_{j}\end{matrix}\right. ,$ wherein hold/gap represents the insertion or deletion of a character, and gap in the formula represents a penalty required by the insertion or deletion of a character (corresponding to a site in a sequence); match means that two characters are the same, and match in the formula represents a bonus when the two characters are the same; mismatch means that two characters are different, and mismatch in the formula represents a penalty when two characters are different. The smallest of the three is taken as *d*[*i, j*]. In one specific example, one gap gives a 3-score penalty, continuous gap gives an additional 1-score penalty, a site mismatch gives a 2-score penalty, and a site match gives 0 score. As such, aligning a sequence containing gaps may be facilitated.

According to one specific embodiment of the present disclosure, the common portion is a common subsequence. According to one specific embodiment of the present disclosure, (d) comprises: searching for a common subsequence of short segments mapped to the same entry of the reference library in the second mapping result to determine the longest common subsequence corresponding to each read; and extending the longest common subsequence based on the edit distance to give an extended sequence.

In one example, for a reconstructed sequence/read, the longest common subsequence of the reconstructed sequence and the reference sequence corresponding to the reconstructed sequence is searched, and based on the longest common subsequence, the reconstructed sequence corresponding to the longest common subsequence is converted into the reference sequence corresponding to the longest common subsequence. The Smith Waterman algorithm can be employed to find out the edit distance between the two sequences. For two strings *x*₁*x*₂*...xᵢ* and *y*₁*y*₂*...yⱼ,* it can be calculated by the following formula: $F \left[i, j\right] = max \left\{\begin{matrix}0 i = 0 or j = 0 \\ F \left(i-1,j-1\right) + σ \left(i, j\right) \\ F \left(i-1,j\right) + σ \left(-,j\right) \\ F \left(i,j-1\right) + σ \left(i,-\right)\end{matrix}\right. ,$ wherein *σ* represents a scoring function, *σ*(*i, j*) represents a score of mismatch and match between characters (sites) *xᵢ* and *yⱼ*, *σ*(-,*j*) represents a score of x*ᵢ* gap (deletion) or y*ⱼ* insertion, and *σ*(*i*, -) represents a score of y*ⱼ* deletion or x*ᵢ* insertion. Then, the method for calculating edit distance in the example above can be employed to convert the reconstructed sequence corresponding to the longest common subsequence into the reference sequence corresponding to the reconstructed sequence. Both termini of the reconstructed sequence corresponding to the longest common subsequence can be extended continuously to find out the minimized character operations (substitution, deletion and insertion).

In one specific example, one gap gives a 3-score penalty, continuous gap gives an additional 1-score penalty, a site mismatch gives a 2-score penalty, and a site match gives 4-score bonus. As such, sequences containing gaps can be aligned efficiently, and sequences containing gaps and having high accuracy at other sites can be kept.

According to one specific embodiment of the present disclosure, (d) further comprises: truncating the extended sequence from at least one end of the extended sequence at a site meeting the following condition: the proportion of the mismatched sites of the truncated extended sequence is less than a third preset value. As such, by truncation and culling, a well-matched partial sequence can be kept, which can help to increase the effective rate of data.

Particularly, according to one specific embodiment of the present disclosure, the extended sequence is truncated based on the following: (i) a first error rate and a second error rate are calculated; if the first error rate is less than the second error rate, then the extended sequence is truncated from a first end of the extended sequence, and if the first error rate is greater than the second error rate, then the extended sequence is truncated from the second end of the extended sequence, thus giving a truncated extended sequence, wherein the first error rate is the proportion of mismatched sites of the truncated extended sequence given by truncating the extended sequence from the first end of the extended sequence, and the second error rate is the proportion of mismatched sites of the truncated extended sequence given by truncating the extended sequence from the second end of the extended sequence; (ii) step (i) is performed with the truncated extended sequence instead of the extended sequence until the proportion of mismatched sites of a truncated extended sequence is less than a fourth preset value. As such, by double-end truncation and culling, a well-matched partial sequence can be kept, which can help to increase the effective rate of data. According to one specific example, the length of the extended sequence is 25 bp, and the fourth preset value is the third preset value, which is 0.12.

According to one specific embodiment of the present disclosure, (d) further comprises: sliding a window on the extended sequence from at least one end of the extended sequence; and truncating the extended sequence according to the proportion of the mismatched sites of the window sequences until the following condition is met: the proportion of mismatched sites of window sequences acquired by sliding is greater than a fifth preset value. As such, by truncation and culling, a well-matched partial sequence can be kept, which can help to increase the effective rate of data.

Particularly, according to one specific embodiment of the present disclosure, the extended sequence is truncated based on the following: (i) a third error rate and a fourth error rate are calculated; if the third error rate is less than the fourth error rate, then the extended sequence is truncated from the second end of the extended sequence, and if the third error rate is greater than the fourth error rate, then the extended sequence is truncated from the first end of the extended sequence, thus giving a truncated extended sequence, wherein the third error rate is the proportion of mismatched sites of the window sequence given by sliding a window on the extended sequence from the first end of the extended sequence, and the fourth error rate is the proportion of mismatched sites of the window sequence given by sliding a window on the extended sequence from the second end of the extended sequence; (ii) step (i) is performed with the truncated extended sequence instead of the extended sequence until the proportion of mismatched sites of the window sequence is less than a sixth preset value. As such, by double-end truncation and culling, a well-matched partial sequence can be kept, which can help to increase the effective rate of data.

According to one specific embodiment of the present disclosure, the size of the sliding window is not greater than the length of the extended sequence. According to one specific example, the length of the extended sequence is 25 bp, the size of the sliding window is 10 bp, and the sixth preset value is the fifth preset value, which is 0.12.

According to one specific embodiment of the present disclosure, the truncated size is 1 bp, that is, 1 base is removed each time during truncation. As such, an alignment result containing more long sequences can be given efficiently.

In order to make a difference comparison result more statistically significant, generally, multiple negative controls are provided. For example, the number of negative controls is not less than 20, preferably not less than 30.

The negative controls are samples without chromosomal aneuploid abnormalities. For example, if the subject of variation testing is a human or the sample under test is a sample from human, the negative control is a sample acquired from a normal diploid individual. The order for acquiring the sequencing result of the negative control and the sequencing result of the sample under test is not limited. For example, they can be acquired simultaneously or successively, and preferably they can be acquired simultaneously under the same experimental conditions in order to decrease the influence of experimental factor difference on testing results as much as possible. In addition, preferably, the negative control and the sample under test are samples of the same type. For example, in order to detect the genetic information of a fetus in a mother in a non-invasive manner, both the negative control and the sample under test are maternal blood samples. According to one specific embodiment of the present disclosure, determining the amount of reads mapped to the corresponding first chromosome in the negative control comprises: subjecting the negative control to steps (1) to (3) instead of the sample under test to determine the amount of reads mapped to the first chromosome in the negative control; and taking the mean of the amount of the reads mapped to the first chromosome in a plurality of negative controls as the amount of the reads mapped to the first chromosome in the negative control.

The amount of reads mapped to the chromosome may be an absolute number or a relative number. For example, the amount may be expressed as a numerical value (such as an integer or a proportion) or a numerical value range.

According to one specific embodiment of the present disclosure, before step (3) is conducted, at least one or two or all of (i) to (iii) below are performed: (i) removing the reads with lengths not greater than a predefined length from the sequencing result; (ii) removing the reads not mapped to a unique location in the first reference sequence from the alignment result, wherein the reads aligned/mapped to unique positions/locations of the reference sequence are referred to as unique reads; (iii) removing the reads with error rates not less than a predefined error rate from the alignment result, wherein the error rate of a read is the ratio of bases of at least one of insertions, deletions and mismatches in the read after alignment.

In one example, the error rate of the read is the ratio of bases or positions of insertions, deletions and mismatches on the read after alignment.

The predetermined error rate may be set according to a sequencing platform, the quantity of off-line data, data quality, the purpose of testing, *etc.* It can be understood that if the quantity of off-line data is small and/or the data quality is high, it may be appropriate to set a high predetermined error rate; otherwise, a low predetermined error rate may be set to remove relatively low-quality data while meeting testing requirement, which is favorable for rapid testing.

In one example, for a sequencing result from a single-molecule sequencing platform, all of steps (i) to (iii) are employed to screen the sequencing result, which is favorable for rapid testing. Particularly, the quantity of off-line data is 12.8 M, the predetermined error rate is set as 10%, *i.e.,* for a 10 bp read, up to 1 bp of insertion, deletion or mismatch is allowed, and after screening, 3.4 M of data is acquired. It can be understood that if a relatively strict screening has been performed during alignment, (ii) may not be performed, for example, the predetermined error rate may be set as 100%.

In one specific embodiment of the present disclosure, step (3) comprises: (a) sliding a window of size L3 on the first reference sequence to give a plurality of third windows, wherein, optionally, the step size of sliding is L3; (b) determining the sequencing depths of the third windows based on the alignment result, wherein the sequencing depth of the third window is the ratio of the number of reads mapping to the third window to the size L3 of the third window; and (c) determining the amount of reads mapped to the first chromosome based on the sequencing depths of the third windows contained in the first chromosome.

According to one specific embodiment, (b) comprises: correcting the sequencing depth of the third window based on GC content of the third window, and taking the corrected sequencing depth of the third window as sequencing depth of the third window.

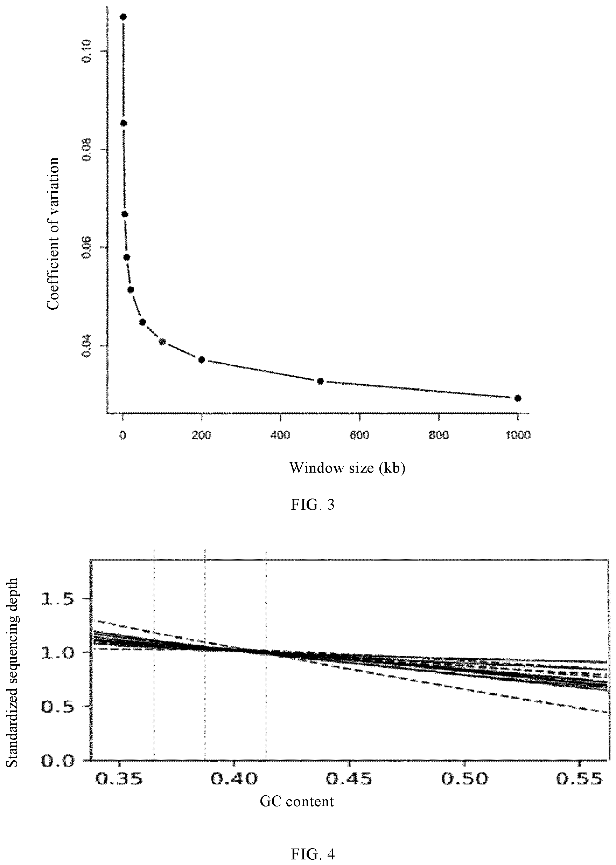

The setting of the size (*i.e.,* L3) of the third windows is generally required to reflect the difference in sequencing results of these regions (the third windows) caused by the difference in GC content and distribution. For a human genome, generally, the value of L3 is less than 300 Kbp. In one example, L3 is determined according to the relationship between coefficients of variation (CV) and windows of different sizes. As shown in FIG. 3, according to the curve, a window size significantly affecting CV is selected as the size of the third window. For example, an L3 set as 100 Kbp to 200 Kbp can reflect the influence of GC content and distribution on sequencing, and is also favorable for rapid alignment. The coefficient of variation (also referred to as a coefficient of dispersion), is a normalized measure for the dispersion of probability distribution and a ratio of standard deviation to mean. It reflects an absolute value of the dispersion of GC contents in a set of windows/regions of a specific window size herein.

Two adjacent third windows may or may not overlap. In one example, L3 is set as 150 Kbp, and two adjacent third windows have a 100 bp overlap, *i.e.,* the step size of sliding is set as 149.9 Kbp.

Particularly, the correction is performed by establishing the relationship between the GC content of the third window and the sequencing depth of the third window. In one example, the relationship between the GC content of the third window and the sequencing depth of the third window is established by locally weighted regression.

According to one specific embodiment, (b) further comprises: standardizing, before the correction is performed, the sequencing depth of the third window, and taking the standardized sequencing depth of the third window as sequencing depth of the third window.

In one example, the standardization is normalization. For example, the sequencing depths of the third windows can be normalized based on the mean or median sequencing depth.

According to one specific embodiment of the present disclosure, in (c), the weight coefficient of a read mapping to the third windows is determined based on the sequencing depth of the third window, and the amount of reads mapped to the first chromosome is determined based on the weight coefficients.

In one example, the sequencing depth of the third window is standardized or normalized. For example, with ratios of the sequencing depths of the third windows to a specific value (a mean sequencing depth of the third window) being the sequencing depths of the third windows, the sequencing depths of the third windows are converted into a set of numerical values fluctuating around 1. The relationship between the processed sequencing depths (relative sequencing depths) and GC contents is determined. The weight coefficient of a read of the third windows is the relative sequencing depth of the window. In one example, the number of the reads mapped to the first chromosome is a relative number which has been corrected by the weight coefficient. Therefore, the influence of GC content and/or distribution differences on the testing result can be eliminated or reduced, thus increasing the accuracy of testing. In some examples, it was discovered that the relative sequencing depth of the third window is inversely proportional to the GC content of the window, *i.e.,* the relative sequencing depth of a third window with low GC content is high, while the relative sequencing depth of a third window with high GC content is low. Thus, for the relative number corrected by the weight coefficient, for example, N reads are mapped to a third window of the first chromosome, the relative sequencing depth of the third window of the first chromosome is w, and then, after correction, ${N *}^{{}^{\frac{1}{w}}}$ reads are mapped to the third window of the first chromosome.

In one example, the amount of reads mapped to the first chromosome is a relative amount, which is a ratio of the amount of reads mapped to the first chromosome and the amount of reads mapped to all or at least part of a normal chromosome. Whether aneuploid abnormality exists in the first chromosome of the sample under test is judged by the statistical significance of the difference between the ratio and the corresponding ratio of the negative controls through z-test (z-score) comparing.

According to one specific embodiment of the present disclosure, the first chromosome is selected from at least one of chromosomes 13, 18 and 21. For example, cell-free nucleic acids in the peripheral blood sample of a pregnant woman is tested to give the genetic information of the fetus, including screening or assisted diagnosis for aneuploidy variation in chromosomes 13, 18 and/or 21 of the fetus.

Generally, GC contents and distributions of different chromosomes have different characteristics. For example, based on the relative GC content, chromosomes in a genome may be classified as high GC content, medium GC content, or low GC content, or may be classified into relatively high GC content, medium-high GC content, medium GC content, medium-low GC content or low GC content.

Table 2 shows GC contents of normal human chromosomes. A curve of relation between the standardized sequencing depth of a chromosome and the GC content of the chromosome was plotted based on sequencing data of multiple reference samples. As shown in FIG. 4, the sequencing results of chromosomes with relatively high and relatively low GC contents are significantly affected by the GC contents. It can be seen from comparison of chromosomes 21, 13 and 18 that the influence of GC content on the sequencing result is minimal in chromosome 21, followed by chromosome 18, and the influence of GC content is the greatest in chromosome 13.

**Table 2**

| | | | | | | | | | | |
|---|---|---|---|---|---|---|---|---|---|---|
| Chr No. | 4 | 5 | 6 | 3 | 18 | 8 | 2 | 7 | 12 | 21 |
| GC content | 0.3825 | 0.3952 | 0.3961 | 0.3969 | 0.3979 | 0.4018 | 0.4024 | 0.4075 | 0.4081 | 0.4083 |
| Chr No. | 14 | 11 | 10 | 1 | 15 | 20 | 16 | 17 | 22 | 19 |
| GC content | 0.4089 | 0.4157 | 0.4158 | 0.4174 | 0.4220 | 0.4413 | 0.4479 | 0.4554 | 0.4799 | 0.4836 |

According to one specific embodiment of the present disclosure, the sample under test is a blood sample from a pregnant woman. As fetal cell-free nucleic acids comprises cell-free fetal DNA (cff DNA), the content in a maternal cell-free nucleic acid sample varies dramatically in different pregnant women and/or in different period of pregnancy. If the sensitivity of the test is increased and the test can be performed at an earlier stage of pregnancy with comparable accuracy, medical intervention may be given at an earlier stage of pregnancy, and less impact may be posed on the pregnant woman. If the accuracy can be increased, the false positive and false negative rates can be reduced, which ultimately makes its application in diagnosis possible in addition to screening for assisting diagnosis. Generally, the body fluid sample of a pregnant woman is subjected to cff DNA extraction, library creation, loading for sequencing and unloading to give sequencing data (*e.g.,* in fastq format). The off-line data is aligned to a reference sequence to give an alignment result (*e.g*., a sam file) including the location of each read on a genome, an alignment score, whether a match is unique or not, alignment errors, and other information. The number of reads of a chromosome in the alignment result can be summarized, and finally, the proportion of reads of the chromosome in reads of the normal chromosome (referred to as chromosome proportion hereinafter) is calculated to judge whether aneuploidy exists in the chromosome.

According to one specific embodiment of the present disclosure, a non-invasive prenatal testing (NIPT, or NIPD, non-invasive prenatal diagnosis) is performed to give a set of body fluid samples (negative controls) of a pregnant woman which contain cell-free DNA and have been confirmed with normal fetus, and the proportion of a chromosome (such as chromosome 21/18/13) in these body fluid samples of the pregnant woman is calculated to determine the range or boundary of normality and/or abnormality for the chromosome or these chromosomes. The range or boundary of normality and/or abnormality of the chromosome can also be determined in the same way using positive samples.

In one embodiment of the present disclosure, a device for detecting chromosomal aneuploidy is provided, which is configured for implementing the method for detecting chromosomal aneuploidy in any of the aforementioned examples or specific embodiments. The device comprises: a sequencing module configured for sequencing at least a portion of a nucleic acid in a sample under test to give a sequencing result including reads; an alignment module configured for aligning the reads from the sequencing module to a first reference sequence to give an alignment result including specific chromosomes to which the reads are mapped, wherein the first reference sequence is a set of regions with an alignment capability of 1 on a reference genome, and the region with an alignment capability of 1 is a region mapped to a unique location on the reference genome; a quantification module configured for determining, for a first chromosome, the amount of reads mapped to the first chromosome based on the alignment result from the alignment module; and a judgment module configured for comparing the number of the reads mapped to the first chromosome from the quantification module with the amount of reads in a negative control mapped to the first chromosome to determine the number of the first chromosome. The aforementioned description of the technical features and effects of the method for detecting chromosomal aneuploidy in any example or specific embodiment of the present disclosure is also applicable to the device in this embodiment of the present disclosure, and will not be repeated hereinafter.

For example, in some embodiments, determining the alignment capability of a region comprises: sliding a first window of size L1 on a reference genome to give a plurality of regions, wherein the step size of sliding, for example, may be set as 1 bp; and aligning the region to the reference genome to calculate the alignment capability of the region based on the number of locations in the reference genome to which the region maps.

In some embodiments, the number of the negative controls is not less than 20 or, preferably, not less than 30.

In some embodiments, the amount of reads mapped to the corresponding first chromosome in the negative control is determined as follows: subjecting the negative control instead of the sample under test to the sequencing module, alignment module and quantification module, so as to determine the amount of reads mapped to the first chromosome in the negative control; and taking the mean of the numbers of the reads mapped to the first chromosome in a plurality of negative controls as the number of the reads mapped to the first chromosome in the negative control.

In some embodiments, the first reference sequence is at least a portion of sequence in a human reference genome hg19 with the regions shown in Table 1 removed.

In some embodiments, the first reference sequence is at least a portion of the reference genome with regions corresponding to a second window meeting the following condition removed: the sequencing depth of the second window is not less than 4 times the mean sequencing depth of all the second windows.

In other embodiments, the first reference sequence is at least a portion of the reference genome with regions corresponding to a second window meeting the following condition removed: the sequencing depth of the second window is not less than 6 times the mean sequencing depth of all the second windows. In some embodiments, the first reference sequence is at least a portion of the reference genome with the regions mapping to the second windows in the reference genome processed as follows: assigning the sequencing depth of the second window at the 98th percentile to the sequencing depths of the second windows over the 98th percentile.

In other embodiments, the sequencing depth of the second window at the 99th percentile is assigned to the sequencing depths of the second windows over the 99th percentile. The second window is acquired by sliding a window of size L2 on the reference genome, and in one example, the step size of the sliding is also L2. The sequencing depth of the second window is the ratio of the number of reads mapping to the second window to the size L2 of the second window. In some embodiments, the device further comprises a filtering module, which is configured for at least one of (i) to (iii) below: (i) removing the reads with lengths not greater than a predefined length from the sequencing result; (ii) removing the reads not mapped to a unique location in the first reference sequence from the alignment result; and (iii) removing the reads with error rates not less than a predefined error rate from the alignment result, wherein the error rate of a read is the ratio of bases of at least one of insertions, deletions and mismatches in the read after alignment. In some embodiments, the quantification module is configured for the following: (a) sliding a window of size L3 on the first reference sequence to give a plurality of third windows; (b) determining the sequencing depths of the third windows based on the alignment result, wherein the sequencing depth of the third window is the ratio of the number of reads mapping to the third window to the size L3 of the third window; and (c) determining the amount of reads mapped to the first chromosome based on the sequencing depths of the third windows contained in the first chromosome.

In some examples, (b) further comprises standardizing the sequencing depth of the third window, and taking the standardized sequencing depth of the third window as sequencing depth of the third window.

In other examples, (b) further comprises correcting the sequencing depth of the third window based on GC content of the third window, and taking the corrected sequencing depth of the third window as sequencing depth of the third window. The sequencing depth of the third window before correction may be the standardized sequencing depth of the third window.

Particularly, the correction is performed utilizing the relationship between the GC content of the third window and the sequencing depth of the third window. In one example, the relationship between the GC content of the third window and the sequencing depth of the third window is established by locally weighted regression.

In some examples, (c) comprises determining the weight coefficients of reads mapping to the third windows based on the sequencing depths of the third windows, and determining the amount of reads mapped to the first chromosome based on the weight coefficients.

In some embodiments, the sample under test is a blood sample from a pregnant woman.

In some embodiments, the first chromosome is at least one of fetal chromosomes 13, 18 and 21. In one embodiment of the present disclosure, a computer-readable storage medium for storing/carrying a program for execution by a computer is provided, wherein the execution of the program comprises completing the method for detecting chromosomal aneuploidy in any of the aforementioned examples or specific embodiments. The aforementioned description of the technical features and effects of the method and/or device for detecting chromosomal aneuploidy in any example or specific embodiment of the present disclosure is also applicable to the computer-readable storage medium in this embodiment of the present disclosure, and will not be repeated herein.

In one embodiment of the present disclosure, a computer program product is also provided, which comprises an instruction, wherein when the program is executed by a computer, the instruction causes the computer to execute the method for detecting chromosomal aneuploidy in any of the aforementioned examples or specific embodiments.

### EXAMPLE

The reference sequence used was a set of regions of a human reference genome that did not comprise the regions shown in Table 1 and met the following conditions: (1) the alignment capability was 1; and (2) regions of a sequencing depth less than 6 times the mean sequencing depth were removed, or the sequencing depth value at 99th percentile had been assigned to the sequencing depths of regions over the 99th percentile.

Both the control sample and the sample under test were processed as follows:
1. sequencing was performed to give off-line data, *i.e.,* a set of reads; and the reads less than 25 bp were removed;
2. an alignment result (sam file) including unique reads (the reads mapping to unique locations of the reference sequence) and locations of these reads on the reference sequence/reference chromosome was obtained;
3. GC correction was performed, which comprised: cutting the reference sequence into windows/regions with a size of 150 Kbp (Bin = 150 K); calculating the sequencing depth of each Bin according to the unique reads, and normalizing the sequencing depth of each Bin to give a normalized sequencing depth; counting the GC content of each Bin; and establishing the relationship between the normalized sequencing depth and the GC content, *e.g*., fitting to give the relationship between the GC content of the Bin and the normalized sequencing depth of the corresponding Bin with the former one as x and the latter one as y;
4. the number of the reads uniquely mapping to the windows/regions in the alignment result was corrected with y as weight coefficients w, which was expressed as that the score or contribution value of the read is 1/w;
5. the corrected number of the unique reads of the chromosome i was determined, which was expressed as the sum of the scores of all the unique reads of the chromosome i ${W}_{i} = \sum \left(\frac{1}{w}\right)$; and
6. the ratio of the sum of the scores of the unique reads of the chromosome i to all normal chromosomes ${Ratio}_{i} = \frac{{W}_{i}}{{\sum }_{j = 1}^{n = 22} {W}_{j}}$ was calculated.

Then, based on the ratios (Ratioᵢ) of the chromosome i of a plurality of control samples, the mean *µ*ᵢ and variance *σᵢ* of the ratios of the chromosome i were determined;
The Z-score of the chromosome i of the sample under test was calculated using a z-test formula $Zscore = \frac{Ratio - μ}{δ}$, and then compared with a threshold to judge whether the number of the chromosome i in the sample under test is abnormal.

If the Z-score of a chromosome of a maternal peripheral blood sample under test is greater than or equal to 3, the difference is statistically significant and it can be considered that three such chromosomes existed in the fetus conceived by the mother.

For the thresholds, the distribution of the ratios of the chromosome i of the plurality of control samples accords with normal distribution or approximately accords with normal distribution, and Z-scores and corresponding levels of confidence can be searched through a z table (normal distribution table). For example, if a level of confidence is 99.97% and the corresponding Z-score is about 3, exceeding this Z-score means that the probability that the sample is not a normal sample is 99.97%, and it can be determined that abnormality exists. Of course, those skilled in the art can set other levels of confidences as desired and then, with corresponding Z-scores as thresholds, determine whether abnormality exists.

The aforementioned method was implemented in eleven samples confirmed positive by karyotyping, and all were detected. The result is shown in Table 3.

**Table 3**

| | No.1 | No.2 | No.3 | No.4 | No.5 | No.6 | No.7 | No.8 | No.9 | No.10 | No.11 |
|---|---|---|---|---|---|---|---|---|---|---|---|
| Positive Type | Trisomy 13 | Trisomy 18 | Trisomy 21 | Trisomy 21 | Trisomy 21 | Trisomy 18 | Trisomy 13 | Trisomy 21 | Trisomy 21 | Trisomy 21 | Trisomy 21 |
| chr13 | 12.46 | / | / | / | / | / | 17.71 | / | / | / | / |
| chr18 | / | 13.50 | / | / | / | 3.52 | / | / | / | / | / |
| chr21 | / | / | 16.75 | 10.38 | 11.66 | / | / | 9.55 | 16.65 | 21.57 | 16.49 |

In the specification, descriptions such as "one embodiment", "some embodiments", "one or some specific embodiments", "one or some examples", "exemplary" or the like, means that a particular feature, structure or characteristic described in reference to the embodiment or example is included in at least one embodiment or example of the present disclosure. In this specification, the schematic description of the aforementioned terms do not necessarily refer to the same embodiment or example. Moreover, the specific features, structures and other characteristics described may be combined in any one or more embodiments or examples in an appropriate manner.

## Claims

1. A method for detecting chromosomal aneuploidy, comprising:
(1) sequencing at least a portion of a nucleic acid in a sample under test to obtain a sequencing result including reads;
(2) aligning the reads to a first reference sequence to obtain an alignment result including specific chromosomes to which the reads are mapped, wherein the first reference sequence is a set of regions with an alignment capability of 1 on a reference genome, and the region with an alignment capability of 1 is defined as a region mapped to a unique location on the reference genome, and the region has a length that is between 0.5 times and 2 times the length of the reads or the average length of the reads;
(3) determining, for a first chromosome, the amount of reads mapped to the first chromosome based on the alignment result; and
(4) comparing the amount of the reads mapped to the first chromosome with the amount of reads from a negative control mapped to the first chromosome to determine the number of the first chromosome.

2. The method according to claim 1, wherein the determination of the alignment capability of the regions comprises:
sliding a first window of size L1 on the reference genome to obtain a plurality of the regions, wherein, optionally, the step size of sliding is 1 bp; and
aligning the region to the reference genome, to calculate the alignment capability of the region based on the number of locations in the reference genome to which the region maps;
optionally, wherein the number of the negative controls is not less than 20, optionally, not less than 30;
further optionally, wherein the amount of reads mapped to the first chromosome in the negative control is determined as follows:
subjecting the negative control to (1) to (3) instead of the sample under test to determine the amount of reads mapped to the first chromosome in the negative control; and
taking the mean of the amount of the reads mapped to the first chromosome in a plurality of negative controls as the amount of the reads mapped to the first chromosome in the negative control;
optionally, wherein the first reference sequence is at least a portion of human reference genome hg19 with the regions in the following table removed:
| Chrom osome No. | Start position | End position | Chrom osome No. | Start position | End position | Chrom osome No. | Start position | End position | Chrom osome No. | Start position | End position |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 121350000 | 121365000 | 4 | 68265000 | 68280000 | 10 | 42585000 | 42600000 | 22 | 18870000 | 18885000 |
| 1 | 121470000 | 121500000 | 5 | 134250000 | 134265000 | 10 | 127575000 | 127590000 | 23 | 58560000 | 58575000 |
| 1 | 142545000 | 142590000 | 6 | 58770000 | 58785000 | 10 | 135495000 | 135510000 | 24 | 6105000 | 6135000 |
| 1 | 142785000 | 142845000 | 6 | 161025000 | 161040000 | 11 | 51570000 | 51600000 | 24 | 9180000 | 9195000 |
| 1 | 142860000 | 142875000 | 7 | 61785000 | 61800000 | 16 | 33945000 | 33975000 | 24 | 9930000 | 10050000 |
| 1 | 142905000 | 142965000 | 7 | 61965000 | 61980000 | 16 | 46380000 | 46440000 | 24 | 10080000 | 10095000 |
| 1 | 143235000 | 143295000 | 8 | 43080000 | 43110000 | 17 | 22245000 | 22260000 | 24 | 13260000 | 13320000 |
| 1 | 143505000 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 133005000 | 133050000 | 8 | 86730000 | 86745000 | 19 | 8850000 | 8865000 | 24 | 28575000 | 28590000 |
| 2 | 162135000 | 162150000 | 9 | 66960000 | 66975000 | 19 | 27720000 | 27750000 | 24 | 28785000 | 28800000 |
| 2 | 209340000 | 209355000 | 9 | 68400000 | 68700000 | 20 | 29625000 | 29640000 | 24 | 58815000 | 58875000 |
| 3 | 196620000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

3. The method according to claim 1 or 2, wherein the first reference sequence is at least a portion of the reference genome with regions corresponding to a second window meeting the following condition removed: the sequencing depth of the second window is not less than 4 times the mean of sequencing depths of all the second windows, and optionally, the sequencing depth of the second window is not less than 6 times the mean of the sequencing depths of all the second windows;
the second window is acquired by sliding a window of size L2 on the reference genome, and optionally, the step size of the sliding is L2; and
the sequencing depth of the second window is the ratio of the number of reads mapping to the second window to the size of the second window; or
wherein the first reference sequence is at least a portion of the reference genome with the regions matching the second windows in the reference genome processed as follows: assigning the sequencing depth of the second window at the 98th percentile to the sequencing depths of the second windows over the 98th percentile, optionally, assigning the sequencing depth of the second window at the 99th percentile to the sequencing depths of the second windows over the 99th percentile;
the second window is acquired by sliding a window of size L2 on the reference genome, and optionally, the step size of the sliding is L2; and
the sequencing depth of the second window is the ratio of the number of reads mapping to the second window to the size L2 of the second window.

4. The method according to any of claims 1 to 3, wherein the method further comprises at least one of the following (i) to (iii) prior to (3):
(i) removing the reads with lengths not greater than a predefined length from the sequencing result;
(ii) removing the reads not mapped to a unique location in the first reference sequence from the alignment result; and
(iii) removing the reads with error rates not less than a predefined error rate from the alignment result, wherein the error rate of a read is the ratio of bases of at least one of insertions, deletions and mismatches in the read after alignment.

5. The method according to any of claims 1 to 4, wherein (3) further comprises:
(a) sliding a window of size L3 on the first reference sequence to obtain a plurality of third windows;
(b) determining the sequencing depths of the third windows based on the alignment result, wherein the sequencing depth of the third window is the ratio of the number of reads mapping to the third window to the size L3 of the third windows; and
(c) determining the amount of reads mapped to the first chromosome based on the sequencing depth of the third windows contained in the first chromosome;
wherein optionally (b) further comprises:
standardizing the sequencing depth of the third window, and taking the standardized sequencing depth of the third window as the sequencing depth of the third window;
wherein further optionally (b) further comprises:
correcting the sequencing depth of the third window based on GC content of the third window, and taking the corrected sequencing depth of the third window as sequencing depth of the third window; wherein optionally the correction is performed utilizing the relationship between the GC content of the third window and the sequencing depth of the third window; and optionally, the relationship between the GC content of the third window and the sequencing depth of the third window is established by locally weighted regression.

6. The method according to claim 5, wherein (c) comprises:
determining a weight coefficient of reads mapping to the third window based on the sequencing depth of the third window; and
determining the amount of reads mapped to the first chromosome based on the weight coefficient.

7. The method according to any of claims 1 to 6, wherein the sample under test is a blood sample from a pregnant woman;
optionally, wherein the first chromosome is at least one of chromosomes 13, 18 and 21 of a fetus.

8. A device for detecting chromosomal aneuploidy, comprising:
a sequencing module, configured for sequencing at least a portion of a nucleic acid in a sample under test to obtain a sequencing result including reads;
an alignment module, configured for aligning the reads from the sequencing module to a first reference sequence to obtain an alignment result including specific chromosomes to which the reads are mapped;
wherein the first reference sequence is a set of regions with an alignment capability of 1 on a reference genome, the region with an alignment capability of 1 is defined as a region mapped to a unique location on the reference genome, and the region has a length that is between 0.5 times and 2 times the length of the reads or the average length of the reads;
a quantification module, configured for determining, for a first chromosome, the amount of reads mapped to the first chromosome based on the alignment result from the alignment module; and
a judgment module, configured for comparing the amount of the reads mapped to the first chromosome from the quantification module with the amount of reads in a negative control mapped to the first chromosome to determine the number of the first chromosome.

9. The device according to claim 8, wherein the determination of the alignment capability of the regions comprises:
sliding a first window of size L1 on the reference genome to obtain a plurality of the regions, wherein, optionally, the step size of sliding is 1 bp; and
aligning the region to the reference genome, to calculate the alignment capability of the region based on the number of locations in the reference genome to which the region maps;
optionally, wherein the number of the negative controls is not less than 20, optionally, not less than 30;
further optionally, wherein the amount of reads mapped to the first chromosome in the negative control are determined as follows:
loading the negative control instead of the sample under test to the sequencing module, the alignment module and the quantification module, so as to determine the amount of reads mapped to the first chromosome in the negative control; and
taking the mean of the amount of the reads mapped to the first chromosome in a plurality of negative controls as the amount of the reads mapped to the first chromosome in the negative control;
optionally, wherein the first reference sequence is at least a portion of sequence in a human reference genome hg19 with the regions in the following table removed:
| Chrom osome No. | Start position | End position | Chrom osome No. | Start position | End position | Chrom osome No. | Start position | End position | Chrom osome No. | Start position | End position |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 121350000 | 121365000 | 4 | 68265000 | 68280000 | 10 | 42585000 | 42600000 | 22 | 18870000 | 18885000 |
| 1 | 121470000 | 121500000 | 5 | 134250000 | 134265000 | 10 | 127575000 | 127590000 | 23 | 58560000 | 58575000 |
| 1 | 142545000 | 142590000 | 6 | 58770000 | 58785000 | 10 | 135495000 | 135510000 | 24 | 6105000 | 6135000 |
| 1 | 142785000 | 142845000 | 6 | 161025000 | 161040000 | 11 | 51570000 | 51600000 | 24 | 9180000 | 9195000 |
| 1 | 142860000 | 142875000 | 7 | 61785000 | 61800000 | 16 | 33945000 | 33975000 | 24 | 9930000 | 10050000 |
| 1 | 142905000 | 142965000 | 7 | 61965000 | 61980000 | 16 | 46380000 | 46440000 | 24 | 10080000 | 10095000 |
| 1 | 143235000 | 143295000 | 8 | 43080000 | 43110000 | 17 | 22245000 | 22260000 | 24 | 13260000 | 13320000 |
| 1 | 143505000 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 133005000 | 133050000 | 8 | 86730000 | 86745000 | 19 | 8850000 | 8865000 | 24 | 28575000 | 28590000 |
| 2 | 162135000 | 162150000 | 9 | 66960000 | 66975000 | 19 | 27720000 | 27750000 | 24 | 28785000 | 28800000 |
| 2 | 209340000 | 209355000 | 9 | 68400000 | 68700000 | 20 | 29625000 | 29640000 | 24 | 58815000 | 58875000 |
| 3 | 196620000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

10. The device according to claim 8 or 9, wherein the first reference sequence is at least a portion of the reference genome with regions corresponding to a second window meeting the following condition removed: the sequencing depth of the second window is not less than 4 times the mean of sequencing depths of all the second windows, and optionally, the sequencing depth of the second window is not less than 6 times the mean of the sequencing depths of all the second windows;
the second window is acquired by sliding a window of size L2 on the reference genome, and optionally, the step size of the sliding is L2; and
the sequencing depth of the second window is the ratio of the number of reads mapping to the second window to the size of the second window; or
wherein the first reference sequence is at least a portion of the reference genome with the regions matching the second windows in the reference genome processed as follows: assigning the sequencing depth of the second window at the 98th percentile to the sequencing depths of the second windows over the 98th percentile, optionally, assigning the sequencing depth of the second window at the 99th percentile to the sequencing depths of the second windows over the 99th percentile;
the second window is acquired by sliding a window of size L2 on the reference genome, and optionally, the step size of the sliding is L2; and
the sequencing depth of the second window is the ratio of the number of reads mapping to the second window to the size L2 of the second window.

11. The device according to any of claims 8 to 10, further comprising a filtering module, which is configured for at least one of (i) to (iii) below:
(i) removing the reads with lengths not greater than a predefined length from the sequencing result;
(ii) removing the reads not mapped to a unique location in the first reference sequence from the alignment result; and
(iii) removing the reads with error rates not less than a predefined error rate from the alignment result, wherein the error rate of a read is the ratio of bases of at least one of insertions, deletions and mismatches in the read after alignment.

12. The device according to any of claims 8 to 11, wherein the quantification module is configured for:
(a) sliding a window of size L3 on the first reference sequence to obtain a plurality of third windows;
(b) determining the sequencing depths of the third windows based on the alignment result, wherein the sequencing depth of the third window is the ratio of the number of reads mapping to the third window to the size L3 of the third windows; and
(c) determining the amount of reads mapped to the first chromosome based on the sequencing depth of the third windows contained in the first chromosome;
wherein optionally (b) further comprises:
standardizing the sequencing depth of the third window, and taking the standardized sequencing depth of the third window as the sequencing depth of the third window;
wherein further optionally (b) further comprises:
correcting the sequencing depth of the third window based on GC content of the third window, and taking the corrected sequencing depth of the third window as sequencing depth of the third window; wherein optionally the correction is performed utilizing the relationship between the GC content of the third window and the sequencing depth of the third window; and optionally, the relationship between the GC content of the third window and the sequencing depth of the third window is established by locally weighted regression.

13. The device according to claim 12, wherein (c) comprises:
determining a weight coefficient of reads mapping to the third window based on the sequencing depth of the third window; and
determining the amount of reads mapped to the first chromosome based on the weight coefficient.

14. The device according to any of claims 8 to 13, wherein the sample under test is a blood sample from a pregnant woman;
optionally, wherein the first chromosome is at least one of chromosomes 13, 18 and 21 of a fetus.

15. A computer program product comprising an instruction, wherein, when the program is executed on the device according to any of claims 8-14 the instruction causes the computer to execute the method according to any of claims 1 to 7.

## Patentansprüche

1. Verfahren zum Nachweisen chromosomaler Aneuploidie, umfassend:
(1) Sequenzieren mindestens eines Teils einer Nukleinsäure in einer zu untersuchenden Probe, um ein Sequenzierungsergebnis zu erhalten, das Reads einschließt;
(2) Ausrichten der Reads an einer ersten Referenzsequenz, um ein Ausrichtungsergebnis zu erhalten, das spezifische Chromosomen einschließt, auf die die Reads abgebildet werden, wobei die erste Referenzsequenz ein Satz von Regionen mit einer Ausrichtungsfähigkeit von 1 auf einem Referenzgenom ist, und die Region mit einer Ausrichtungsfähigkeit von 1 als eine Region definiert ist, die auf eine eindeutige Position im Referenzgenom abgebildet ist, und die Region eine Länge aufweist, die zwischen dem 0,5-Fachen und dem 2-Fachen der Länge der Reads oder der durchschnittlichen Länge der Reads liegt;
(3) Bestimmen der Anzahl der auf das erste Chromosom abgebildeten Reads für ein erstes Chromosom auf Grundlage des Ausrichtungsergebnisses; und
(4) Vergleichen der Anzahl der Reads, die auf das erste Chromosom abgebildet sind, mit der Anzahl der Reads einer Negativkontrolle, die auf das erste Chromosom abgebildet sind, zum Bestimmen der Anzahl des ersten Chromosoms.

2. Verfahren nach Anspruch 1, wobei das Bestimmen der Ausrichtungsfähigkeit der Regionen umfasst:
Verschieben eines ersten Fensters der Größe L1 auf dem Referenzgenom, um eine Vielzahl von den Regionen zu erhalten, wobei, optional, die Schrittgröße des Verschiebens 1 bp beträgt; und
Ausrichten der Region an dem Referenzgenom, um die Ausrichtungsfähigkeit der Region basierend auf der Anzahl von Positionen im Referenzgenom zu berechnen, auf die die Region abgebildet wird;
wobei optional die Anzahl der Negativkontrollen mindestens 20 beträgt, optional mindestens 30;
ferner optional, wobei die Anzahl der auf das erste Chromosom in der Negativkontrolle abgebildeten Reads wie folgt bestimmt wird:
Unterziehen der Negativkontrolle anstelle der zu untersuchenden Probe den Schritten (1) bis (3), um die Anzahl der auf das erste Chromosom in der Negativkontrolle abgebildeten Reads zu ermitteln; und
Bilden des Mittelwerts der Anzahl der auf das erste Chromosom abgebildeten Reads in einer Vielzahl von Negativkontrollen als Anzahl der auf das erste Chromosom in der Negativkontrolle abgebildeten Reads; wobei optional die erste Referenzsequenz mindestens ein Teil des menschlichen Referenzgenoms hg19 ist, wobei die in der folgenden Tabelle aufgeführten Regionen entfernt sind:
| Chromosom Nr. | Startposition | Endposition | Chromosom Nr. | Startposition | Endposition | Chromosom Nr. | Startposition | Endposition | Chromosom Nr. | Startposition | Endposition |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 121350000 | 121365000 | 4 | 68265000 | 68280000 | 10 | 42585000 | 42600000 | 22 | 18870000 | 18885000 |
| 1 | 121470000 | 121500000 | 5 | 134250000 | 134265000 | 10 | 127575000 | 127590000 | 23 | 58560000 | 58575000 |
| 1 | 142545000 | 142590000 | 6 | 58770000 | 58785000 | 10 | 135495000 | 135510000 | 24 | 6105000 | 6135000 |
| 1 | 142785000 | 142845000 | 6 | 161025000 | 161040000 | 11 | 51570000 | 51600000 | 24 | 9180000 | 9195000 |
| 1 | 142860000 | 142875000 | 7 | 61785000 | 61800000 | 16 | 33945000 | 33975000 | 24 | 9930000 | 10050000 |
| 1 | 142905000 | 142965000 | 7 | 61965000 | 61980000 | 16 | 46380000 | 46440000 | 24 | 10080000 | 10095000 |
| 1 | 143235000 | 143295000 | 8 | 43080000 | 43110000 | 17 | 22245000 | 22260000 | 24 | 13260000 | 13320000 |
| 1 | 143505000 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 133005000 | 133050000 | 8 | 86730000 | 86745000 | 19 | 8850000 | 8865000 | 24 | 28575000 | 28590000 |
| 2 | 162135000 | 162150000 | 9 | 66960000 | 66975000 | 19 | 27720000 | 27750000 | 24 | 28785000 | 28800000 |
| 2 | 209340000 | 209355000 | 9 | 68400000 | 68700000 | 20 | 29625000 | 29640000 | 24 | 58815000 | 58875000 |
| 3 | 196620000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

3. Verfahren nach Anspruch 1 oder 2, wobei die erste Referenzsequenz mindestens ein Teil des Referenzgenoms ist, wobei Regionen entfernt sind, die einem zweiten Fenster entsprechen, welches die folgende Bedingung erfüllt: die Sequenzierungstiefe des zweiten Fensters beträgt mindestens das Vierfache des Mittelwerts der Sequenzierungstiefen aller zweiten Fenster, und optional mindestens das Sechsfache des Mittelwerts der Sequenzierungstiefen aller zweiten Fenster;
das zweite Fenster durch Verschieben eines Fensters der Größe L2 über das Referenzgenom erhalten wird, und optional die Schrittgröße des Verschiebens L2 ist; und
die Sequenzierungstiefe des zweiten Fensters als Verhältnis der Anzahl der auf das zweite Fenster abgebildeten Reads zur Größe des zweiten Fensters bestimmt ist; oder
wobei die erste Referenzsequenz mindestens ein Teil des Referenzgenoms ist, wobei die Regionen, die mit den zweiten Fenstern im Referenzgenom übereinstimmen, wie folgt verarbeitet werden: Zuweisen der Sequenzierungstiefe des zweiten Fensters beim 98. Perzentil zu den Sequenzierungstiefen der zweiten Fenster über dem 98. Perzentil, optional Zuweisen der Sequenzierungstiefe des zweiten Fensters beim 99. Perzentil zu den Sequenzierungstiefen der zweiten Fenster über dem 99. Perzentil;
das zweite Fenster durch Verschieben eines Fensters der Größe L2 über das Referenzgenom erhalten wird, und optional die Schrittgröße des Verschiebens L2 ist; und
die Sequenzierungstiefe des zweiten Fensters als Verhältnis der Anzahl der auf das zweite Fenster abgebildeten Reads zur Größe L2 des zweiten Fensters bestimmt ist.

4. Verfahren nach einem der Ansprüche 1 bis 3, wobei das Verfahren ferner vor Schritt (3) mindestens einen der folgenden Schritte (i) bis (iii) umfasst:
(i) Entfernen der Reads, deren Länge nicht größer als eine vorgegebene Länge ist, aus dem Sequenzierungsergebnis;
(ii) Entfernen der Reads, die nicht auf eine eindeutige Position in der ersten Referenzsequenz abgebildet sind, aus dem Ausrichtungsergebnis; und
(iii) Entfernen der Reads mit Fehlerraten von mindestens einer vorgegebenen Fehlerrate aus dem Ausrichtungsergebnis, wobei die Fehlerrate eines Reads das Verhältnis von Basen von mindestens einem von Insertionen, Deletionen und Fehlpaarungen in dem Read nach der Ausrichtung ist.

5. Verfahren nach einem der Ansprüche 1 bis 4, wobei (3) ferner umfasst:
(a) Verschieben eines Fensters der Größe L3 entlang der ersten Referenzsequenz, um eine Vielzahl von dritten Fenstern zu erhalten;
(b) Bestimmen der Sequenzierungstiefen der dritten Fenster basierend auf dem Ausrichtungsergebnis, wobei die Sequenzierungstiefe des dritten Fensters das Verhältnis der Anzahl von Reads, die auf das dritte Fenster abgebildet werden, zu der Größe L3 der dritten Fenster ist; und
(c) Bestimmen der Anzahl der auf das erste Chromosom abgebildeten Reads auf Grundlage der Sequenzierungstiefe der im ersten Chromosom enthaltenen dritten Fenster;
wobei optional (b) ferner umfasst:
Standardisieren der Sequenzierungstiefe des dritten Fensters und Verwenden der standardisierten Sequenzierungstiefe des dritten Fensters als die Sequenzierungstiefe des dritten Fensters;
wobei ferner optional (b) ferner umfasst:
Korrigieren der Sequenzierungstiefe des dritten Fensters basierend auf dem GC-Gehalt des dritten Fensters und Verwenden der korrigierten Sequenzierungstiefe des dritten Fensters als Sequenzierungstiefe des dritten Fensters; wobei optional die Korrektur optional unter Verwendung der Beziehung zwischen dem GC-Gehalt des dritten Fensters und der Sequenzierungstiefe des dritten Fensters erfolgt; und wobei optional die Beziehung zwischen dem GC-Gehalt des dritten Fensters und der Sequenzierungstiefe des dritten Fensters durch lokal gewichtete Regression hergestellt wird.

6. Verfahren nach Anspruch 5, wobei (c) umfasst:
Bestimmen eines Gewichtungskoeffizienten für die auf das dritte Fenster abgebildeten Reads auf Grundlage der Sequenzierungstiefe des dritten Fensters; und
Bestimmen der Anzahl der auf das erste Chromosom abgebildeten Reads auf Grundlage des Gewichtungskoeffizienten.

7. Verfahren nach einem der Ansprüche 1 bis 6, wobei die zu untersuchende Probe eine Blutprobe von einer schwangeren Frau ist;
wobei optional das erste Chromosom mindestens eines der Chromosomen 13, 18 und 21 eines Fötus ist.

8. Vorrichtung zum Nachweisen chromosomaler Aneuploidie, umfassend:
ein Sequenzierungsmodul, konfiguriert zum Sequenzieren mindestens eines Teils einer Nukleinsäure in einer zu untersuchenden Probe, um ein Sequenzierungsergebnis zu erhalten, das Reads einschließt;
ein Ausrichtungsmodul, konfiguriert zum Ausrichten der Reads aus dem Sequenzierungsmodul auf eine erste Referenzsequenz, um ein Ausrichtungsergebnis zu erhalten, das spezifische Chromosomen einschließt, auf welche die Reads abgebildet sind;
wobei die erste Referenzsequenz ein Satz von Regionen mit einer Ausrichtungsfähigkeit von 1 auf einem Referenzgenom ist, die Region mit einer Ausrichtungsfähigkeit von 1 als eine Region definiert ist, die auf eine eindeutige Position auf dem Referenzgenom abgebildet wird, und die Region eine Länge aufweist, die zwischen dem 0,5-Fachen und dem 2-Fachen der Länge der Reads oder der durchschnittlichen Länge der Reads liegt;
ein Quantifizierungsmodul, konfiguriert zum Bestimmen der Anzahl der auf das erste Chromosom abgebildeten Reads auf Grundlage des Ausrichtungsergebnisses des Ausrichtungsmoduls; und ein Beurteilungsmodul, konfiguriert zum Vergleichen der Anzahl der auf das erste Chromosom abgebildeten Reads, die vom Quantifizierungsmodul bereitgestellt werden, mit der Anzahl der auf das erste Chromosom in einer Negativkontrolle abgebildeten Reads, zum Bestimmen der Anzahl des ersten Chromosoms.

9. Vorrichtung nach Anspruch 8, wobei das Bestimmen der Ausrichtungsfähigkeit der Regionen umfasst:
Verschieben eines ersten Fensters der Größe L1 auf dem Referenzgenom, um eine Vielzahl von den Regionen zu erhalten, wobei, optional, die Schrittgröße des Verschiebens 1 bp beträgt; und
Ausrichten der Region an dem Referenzgenom, um die Ausrichtungsfähigkeit der Region basierend auf der Anzahl von Positionen im Referenzgenom zu berechnen, auf die die Region abgebildet wird;
wobei optional die Anzahl der Negativkontrollen mindestens 20 beträgt, optional mindestens 30;
ferner optional, wobei die Anzahl der auf das erste Chromosom in der Negativkontrolle abgebildeten Reads wie folgt bestimmt wird:
Einbringen der Negativkontrolle anstelle der zu untersuchenden Probe in das Sequenzierungsmodul, das Ausrichtungsmodul und das Quantifizierungsmodul, zum Bestimmen der Anzahl der auf das erste Chromosom in der Negativkontrolle abgebildeten Reads; und
Bilden des Mittelwerts der Anzahl der auf das erste Chromosom abgebildeten Reads in einer Vielzahl von Negativkontrollen als Anzahl der auf das erste Chromosom in der Negativkontrolle abgebildeten Reads; wobei optional die erste Referenzsequenz mindestens ein Teil einer Sequenz eines menschlichen Referenzgenoms hg19 ist, wobei die in der folgenden Tabelle aufgeführten Regionen entfernt sind:
| Chromosom Nr. | Startposition | Endposition | Chromosom Nr. | Startposition | Endposition | Chromosom Nr. | Startposition | Endposition | Chromosom Nr. | Startposition | Endposition |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 121350000 | 121365000 | 4 | 68265000 | 68280000 | 10 | 42585000 | 42600000 | 22 | 18870000 | 18885000 |
| 1 | 121470000 | 121500000 | 5 | 134250000 | 134265000 | 10 | 127575000 | 127590000 | 23 | 58560000 | 58575000 |
| 1 | 142545000 | 142590000 | 6 | 58770000 | 58785000 | 10 | 135495000 | 135510000 | 24 | 6105000 | 6135000 |
| 1 | 142785000 | 142845000 | 6 | 161025000 | 161040000 | 11 | 51570000 | 51600000 | 24 | 9180000 | 9195000 |
| 1 | 142860000 | 142875000 | 7 | 61785000 | 61800000 | 16 | 33945000 | 33975000 | 24 | 9930000 | 10050000 |
| 1 | 142905000 | 142965000 | 7 | 61965000 | 61980000 | 16 | 46380000 | 46440000 | 24 | 10080000 | 10095000 |
| 1 | 143235000 | 143295000 | 8 | 43080000 | 43110000 | 17 | 22245000 | 22260000 | 24 | 13260000 | 13320000 |
| 1 | 143505000 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 133005000 | 133050000 | 8 | 86730000 | 86745000 | 19 | 8850000 | 8865000 | 24 | 28575000 | 28590000 |
| 2 | 162135000 | 162150000 | 9 | 66960000 | 66975000 | 19 | 27720000 | 27750000 | 24 | 28785000 | 28800000 |
| 2 | 209340000 | 209355000 | 9 | 68400000 | 68700000 | 20 | 29625000 | 29640000 | 24 | 58815000 | 58875000 |
| 3 | 196620000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

10. Vorrichtung nach Anspruch 8 oder 9, wobei die erste Referenzsequenz mindestens ein Teil des Referenzgenoms ist, wobei Regionen entfernt sind, die einem zweiten Fenster entsprechen, welches die folgende Bedingung erfüllt: die Sequenzierungstiefe des zweiten Fensters beträgt mindestens das Vierfache des Mittelwerts der Sequenzierungstiefen aller zweiten Fenster, und optional mindestens das Sechsfache des Mittelwerts der Sequenzierungstiefen aller zweiten Fenster;
das zweite Fenster durch Verschieben eines Fensters der Größe L2 über das Referenzgenom erhalten wird, und optional die Schrittgröße des Verschiebens L2 ist; und
die Sequenzierungstiefe des zweiten Fensters als Verhältnis der Anzahl der auf das zweite Fenster abgebildeten Reads zur Größe des zweiten Fensters bestimmt ist; oder
wobei die erste Referenzsequenz mindestens ein Teil des Referenzgenoms ist, wobei die Regionen, die mit den zweiten Fenstern im Referenzgenom übereinstimmen, wie folgt verarbeitet werden: Zuweisen der Sequenzierungstiefe des zweiten Fensters beim 98. Perzentil zu den Sequenzierungstiefen der zweiten Fenster über dem 98. Perzentil, optional Zuweisen der Sequenzierungstiefe des zweiten Fensters beim 99. Perzentil zu den Sequenzierungstiefen der zweiten Fenster über dem 99. Perzentil;
das zweite Fenster durch Verschieben eines Fensters der Größe L2 über das Referenzgenom erhalten wird, und optional die Schrittgröße des Verschiebens L2 ist; und
die Sequenzierungstiefe des zweiten Fensters als Verhältnis der Anzahl der auf das zweite Fenster abgebildeten Reads zur Größe L2 des zweiten Fensters bestimmt ist.

11. Vorrichtung nach einem der Ansprüche 8 bis 10, ferner umfassend ein Filtermodul, das für mindestens einen der folgenden Schritte (i) bis (iii) konfiguriert ist:
(i) Entfernen der Reads, deren Länge nicht größer als eine vorgegebene Länge ist, aus dem Sequenzierungsergebnis;
(ii) Entfernen der Reads, die nicht auf eine eindeutige Position in der ersten Referenzsequenz abgebildet sind, aus dem Ausrichtungsergebnis; und
(iii) Entfernen der Reads mit Fehlerraten von mindestens einer vorgegebenen Fehlerrate aus dem Ausrichtungsergebnis, wobei die Fehlerrate eines Reads das Verhältnis von Basen von mindestens einem von Insertionen, Deletionen und Fehlpaarungen in dem Read nach der Ausrichtung ist.

12. Vorrichtung nach einem der Ansprüche 8 bis 11, wobei das Quantifizierungsmodul konfiguriert ist zum:
(a) Verschieben eines Fensters der Größe L3 entlang der ersten Referenzsequenz, um eine Vielzahl von dritten Fenstern zu erhalten;
(b) Bestimmen der Sequenzierungstiefen der dritten Fenster basierend auf dem Ausrichtungsergebnis, wobei die Sequenzierungstiefe des dritten Fensters das Verhältnis der Anzahl von Reads, die auf das dritte Fenster abgebildet werden, zu der Größe L3 der dritten Fenster ist; und
(c) Bestimmen der Anzahl der auf das erste Chromosom abgebildeten Reads auf Grundlage der Sequenzierungstiefe der im ersten Chromosom enthaltenen dritten Fenster;
wobei optional (b) ferner umfasst:
Standardisieren der Sequenzierungstiefe des dritten Fensters und Verwenden der standardisierten Sequenzierungstiefe des dritten Fensters als die Sequenzierungstiefe des dritten Fensters;
wobei ferner optional (b) ferner umfasst:
Korrigieren der Sequenzierungstiefe des dritten Fensters basierend auf dem GC-Gehalt des dritten Fensters und Verwenden der korrigierten Sequenzierungstiefe des dritten Fensters als Sequenzierungstiefe des dritten Fensters; wobei optional die Korrektur optional unter Verwendung der Beziehung zwischen dem GC-Gehalt des dritten Fensters und der Sequenzierungstiefe des dritten Fensters erfolgt; und wobei optional die Beziehung zwischen dem GC-Gehalt des dritten Fensters und der Sequenzierungstiefe des dritten Fensters durch lokal gewichtete Regression hergestellt wird.

13. Vorrichtung nach Anspruch 12, wobei (c) umfasst:
Bestimmen eines Gewichtungskoeffizienten für die auf das dritte Fenster abgebildeten Reads auf Grundlage der Sequenzierungstiefe des dritten Fensters; und
Bestimmen der Anzahl der auf das erste Chromosom abgebildeten Reads auf Grundlage des Gewichtungskoeffizienten.

14. Vorrichtung nach einem der Ansprüche 8 bis 13, wobei die zu untersuchende Probe eine Blutprobe von einer schwangeren Frau ist;
wobei optional das erste Chromosom mindestens eines der Chromosomen 13, 18 und 21 eines Fötus ist.

15. Computerprogrammprodukt, umfassend eine Anweisung, wobei, wenn das Programm auf der Vorrichtung nach einem der Ansprüche 8 bis 14 ausgeführt wird, die Anweisung den Computer veranlasst, das Verfahren nach einem der Ansprüche 1 bis 7 auszuführen.

## Revendications

1. Procédé permettant de détecter une aneuploïdie chromosomique, comprenant :
(1) le séquençage d'au moins une partie d'un acide nucléique dans un échantillon soumis à un test pour obtenir un résultat de séquençage comportant des lectures ;
(2) l'alignement des lectures sur une première séquence de référence pour obtenir un résultat d'alignement comportant des chromosomes spécifiques avec lesquels les lectures sont mises en correspondance, dans lequel la première séquence de référence est un ensemble de régions avec une capacité d'alignement de 1 sur un génome de référence, et la région avec une capacité d'alignement de 1 est définie en tant que région mise en correspondance avec une localisation unique sur le génome de référence, et la région a une longueur qui est comprise entre 0,5 fois et 2 fois la longueur des lectures ou la longueur moyenne des lectures ;
(3) la détermination, pour un premier chromosome, de la quantité de lectures mises en correspondance avec le premier chromosome en fonction du résultat d'alignement ; et
(4) la comparaison de la quantité des lectures mises en correspondance avec le premier chromosome à la quantité de lectures provenant d'un témoin négatif mises en correspondance avec le premier chromosome pour déterminer le numéro du premier chromosome.

2. Procédé selon la revendication 1, dans lequel la détermination de la capacité d'alignement des régions comprend :
le glissement d'une première fenêtre de taille L1 sur le génome de référence pour obtenir une pluralité des régions, dans lequel, facultativement, la taille de pas de glissement est de 1 bp ; et
l'alignement de la région avec le génome de référence, pour calculer la capacité d'alignement de la région en fonction du nombre de localisations dans le génome de référence avec lesquelles la région se met en correspondance ;
facultativement, dans lequel le nombre des témoins négatifs n'est pas inférieur à 20, facultativement, pas inférieur à 30 ;
facultativement en outre, dans lequel la quantité de lectures mises en correspondance avec le premier chromosome dans le témoin négatif est déterminée comme suit :
la soumission du témoin négatif à (1) à (3) au lieu de l'échantillon soumis à un test pour déterminer la quantité de lectures mises en correspondance avec le premier chromosome dans le témoin négatif ; et
le fait de prendre la moyenne arithmétique de la quantité des lectures mises en correspondance avec le premier chromosome dans une pluralité de témoins négatifs en guise de quantité des lectures mises en correspondance avec le premier chromosome dans le témoin négatif ; facultativement, dans lequel la première séquence de référence est au moins une partie de génome de référence humain hg19 avec les régions dans le tableau suivant retirées :
| Chromosome No. | Position de départ | Position de fin | Chromosome No. | Position de départ | Position de fin | Chromosome No. | Position de départ | Position de fin | Chromosome No. | Position de départ | Position de fin |
|---|---|---|---|---|---|---|---|---|---|---|---|
| 1 | 555000 | 570000 | 4 | 49275000 | 49335000 | 10 | 42375000 | 42405000 | 22 | 18660000 | 186900 |
| 1 | 91845000 | 91860000 | 4 | 52650000 | 52665000 | 10 | 42525000 | 42540000 | 22 | 18720000 | 187350 |
| 1 | 121350000 | 121365000 | 4 | 68265000 | 68280000 | 10 | 42585000 | 42600000 | 22 | 18870000 | 188850 |
| 1 | 121470000 | 121500000 | 5 | 134250000 | 134265000 | 10 | 12757500 | 12759000 | 23 | 58560000 | 585750 |
| 1 | 142545000 | 142590000 | 6 | 58770000 | 58785000 | 10 | 13549500 | 13551000 | 24 | 6105000 | 613500 |
| 1 | 142785000 | 142845000 | 6 | 161025000 | 161040000 | 11 | 51570000 | 51600000 | 24 | 9180000 | 919500 |
| 1 | 142860000 | 142875000 | 7 | 61785000 | 61800000 | 16 | 33945000 | 33975000 | 24 | 9930000 | 100500 |
| 1 | 142905000 | 142965000 | 7 | 61965000 | 61980000 | 16 | 46380000 | 46440000 | 24 | 10080000 | 100950 |
| 1 | 143235000 | 143295000 | 8 | 43080000 | 43110000 | 17 | 22245000 | 22260000 | 24 | 13260000 | 133200 |
| 1 | 143505000 | 143520000 | 8 | 43785000 | 43800000 | 17 | 45210000 | 45225000 | 24 | 13395000 | 135000 |
| 2 | 90375000 | 90390000 | 8 | 43815000 | 43830000 | 18 | 105000 | 120000 | 24 | 13635000 | 137100 |
| 2 | 92265000 | 92325000 | 8 | 86550000 | 86565000 | 18 | 18510000 | 18525000 | 24 | 13800000 | 138750 |
| 2 | 133005000 | 133050000 | | 86730000 | 86745000 | 19 | 8850000 | 8865000 | 24 | 28575000 | 285900 |
| 2 | 162135000 | 162150000 | 9 | 66960000 | 66975000 | 19 | 27720000 | 27750000 | 24 | 28785000 | 288000 |
| 2 | 209340000 | 209355000 | 9 | 38400000 | 68700000 | 20 | 29625000 | 29640000 | 24 | 58815000 | 588750 |
| 3 | 196620000 | 196635000 | 9 | 68685000 | 68730000 | 21 | 9825000 | 9840000 | 24 | 58965000 | 590400 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

3. Procédé selon la revendication 1 ou 2, dans lequel la première séquence de référence est au moins une partie du génome de référence, avec des régions correspondant à une deuxième fenêtre respectant la condition suivante, retirées : la profondeur de séquençage de la deuxième fenêtre n'est pas inférieure à 4 fois la moyenne arithmétique des profondeurs de séquençage de toutes les deuxièmes fenêtres, et facultativement, la profondeur de séquençage de la deuxième fenêtre n'est pas inférieure à 6 fois la moyenne arithmétique des profondeurs de séquençage de toutes les deuxièmes fenêtres ;
la deuxième fenêtre est acquise par glissement d'une fenêtre de taille L2 sur le génome de référence, et facultativement, la taille de pas du glissement est L2 ; et
la profondeur de séquençage de la deuxième fenêtre est le rapport du nombre de lectures se mettant en correspondance avec la deuxième fenêtre à la taille de la deuxième fenêtre ; ou
dans lequel la première séquence de référence est au moins une partie du génome de référence avec les régions concordant avec les deuxièmes fenêtres dans le génome de référence traitées comme suit : attribution de la profondeur de séquençage de la deuxième fenêtre au 98e centile aux profondeurs de séquençage des deuxièmes fenêtres au-delà du 98e centile, facultativement, attribution de la profondeur de séquençage de la deuxième fenêtre au 99e centile aux profondeurs de séquençage des deuxièmes fenêtres au-delà du 99e centile ;
la deuxième fenêtre est acquise par glissement d'une fenêtre de taille L2 sur le génome de référence, et facultativement, la taille de pas du glissement est L2 ; et
la profondeur de séquençage de la deuxième fenêtre est le rapport du nombre de lectures se mettant en correspondance avec la deuxième fenêtre à la taille L2 de la deuxième fenêtre.

4. Procédé selon l'une quelconque des revendications 1 à 3, dans lequel le procédé comprend en outre au moins l'un des points (i) à (iii) qui suivent avant le point (3) :
(i) le fait de retirer du résultat de séquençage des lectures avec des longueurs ne dépassant pas une longueur prédéfinie ;
(ii) le fait de retirer du résultat d'alignement les lectures non mises en correspondance avec une localisation unique dans la première séquence de référence ; et
(iii) le fait de retirer du résultat d'alignement les lectures avec des taux d'erreur pas inférieurs à un taux d'erreur prédéfini, dans lequel le taux d'erreur d'une lecture est le rapport des bases d'au moins l'une parmi des insertions, des délétions et des discordances dans la lecture après alignement.

5. Procédé selon l'une quelconque des revendications 1 à 4, dans lequel (3) comprend en outre :
(a) le glissement d'une fenêtre de taille L3 sur la première séquence de référence pour obtenir une pluralité de troisièmes fenêtres ;
(b) la détermination des profondeurs de séquençage des troisièmes fenêtres en fonction du résultat d'alignement, dans lequel la profondeur de séquençage de la troisième fenêtre est le rapport du nombre de lectures se mettant en correspondance avec la troisième fenêtre à la taille L3 des troisièmes fenêtres ; et
(c) la détermination de la quantité de lectures mises en correspondance avec le premier chromosome en fonction de la profondeur de séquençage des troisièmes fenêtres contenues dans le premier chromosome ;
dans lequel facultativement le point (b) comprend en outre :
la normalisation de la profondeur de séquençage de la troisième fenêtre, et le fait de prendre la profondeur de séquençage normalisée de la troisième fenêtre en guise de profondeur de séquençage de la troisième fenêtre ;
dans lequel facultativement en outre le point (b) comprend en outre :
la correction de la profondeur de séquençage de la troisième fenêtre en fonction d'une teneur en GC de la troisième fenêtre, et le fait de prendre la profondeur de séquençage corrigée de la troisième fenêtre en tant que profondeur de séquençage de la troisième fenêtre ; dans lequel facultativement la correction est mise en œuvre en utilisant la relation entre la teneur en GC de la troisième fenêtre et la profondeur de séquençage de la troisième fenêtre ; et facultativement, la relation entre la teneur en GC de la troisième fenêtre et la profondeur de séquençage de la troisième fenêtre est établie par régression localement pondérée.

6. Procédé selon la revendication 5, dans lequel le point (c) comprend :
la détermination d'un coefficient de pondération de lectures se mettant en correspondance avec la troisième fenêtre en fonction de la profondeur de séquençage de la troisième fenêtre ; et
la détermination de la quantité de lectures mises en correspondance avec le premier chromosome en fonction du coefficient de pondération.

7. Procédé selon l'une quelconque des revendications 1 à 6, dans lequel l'échantillon soumis à un test est un échantillon de sang provenant d'une femme enceinte ;
facultativement, dans lequel le premier chromosome est au moins l'un des chromosomes 13, 18 et 21 d'un fœtus.

8. Dispositif permettant de détecter une aneuploïdie chromosomique, comprenant :
un module de séquençage, configuré pour le séquençage d'au moins une partie d'un acide nucléique dans un échantillon soumis à un test pour obtenir un résultat de séquençage comportant des lectures ;
un module d'alignement, configuré pour l'alignement des lectures provenant du module de séquençage avec une première séquence de référence pour obtenir un résultat d'alignement comportant des chromosomes spécifiques avec lesquels les lectures sont mises en correspondance ;
dans lequel la première séquence de référence est un ensemble de régions avec une capacité d'alignement de 1 sur un génome de référence, la région avec une capacité d'alignement de 1 est définie en tant que région mise en correspondance avec une localisation unique sur le génome de référence, et la région a une longueur qui est comprise entre 0,5 fois et 2 fois la longueur des lectures ou la longueur moyenne des lectures ;
un module de quantification, configuré pour la détermination, pour un premier chromosome, de la quantité de lectures mises en correspondance avec le premier chromosome en fonction du résultat d'alignement provenant du module d'alignement ; et un module d'évaluation, configuré pour la comparaison de la quantité des lectures mises en correspondance avec le premier chromosome provenant du module de quantification à la quantité de lectures dans un témoin négatif mises en correspondance avec le premier chromosome pour déterminer le numéro du premier chromosome.

9. Dispositif selon la revendication 8, dans lequel la détermination de la capacité d'alignement des régions comprend :
le glissement d'une première fenêtre de taille L1 sur le génome de référence pour obtenir une pluralité des régions, dans lequel, facultativement, la taille de pas de glissement est de 1 bp ; et
l'alignement de la région avec le génome de référence, pour calculer la capacité d'alignement de la région en fonction du nombre de localisations dans le génome de référence avec lesquelles la région se met en correspondance ;
facultativement, dans lequel le nombre des témoins négatifs n'est pas inférieur à 20, facultativement, pas inférieur à 30 ;
facultativement en outre, dans lequel la quantité de lectures mises en correspondance avec le premier chromosome dans le témoin négatif est déterminée comme suit :
le chargement du témoin négatif au lieu de l'échantillon soumis à un test dans le module de séquençage, le module d'alignement et le module de quantification, de façon à déterminer la quantité de lectures mises en correspondance avec le premier chromosome dans le témoin négatif ; et
le fait de prendre la moyenne arithmétique de la quantité des lectures mises en correspondance avec le premier chromosome dans une pluralité de témoins négatifs en guise de quantité des lectures mises en correspondance avec le premier chromosome dans le témoin négatif ; facultativement, dans lequel la première séquence de référence est au moins une partie de séquence dans un génome de référence humain hg19 avec les régions dans le tableau suivant retirées :
| Chromosome No. | Position de départ | Position de fin | Chromosome No. | Position de départ | Position de fin | Chromosome No. | Position de départ | Position de fin | Chromosome No. | Position de départ | Position de fin |
|---|---|---|---|---|---|---|---|---|---|---|---|
| 1 | 555000 | 570000 | 4 | 49275000 | 49335000 | 10 | 42375000 | 42405000 | 22 | 18660000 | 18690000 |
| 1 | 91845000 | 91860000 | 4 | 52650000 | 52665000 | 10 | 42525000 | 42540000 | 22 | 18720000 | 18735000 |
| 1 | 12135000 | 121365000 | 4 | 68265000 | 68280000 | 10 | 42585000 | 42600000 | 22 | 18870000 | 18885000 |
| 1 | 12147000 | 121500000 | 5 | 134250000 | 134265000 | 10 | 127575000 | 127590000 | 23 | 58560000 | 58575000 |
| 1 | 14254500 | 142590000 | 6 | 58770000 | 58785000 | 10 | 135495000 | 135510000 | 24 | 6105000 | 6135000 |
| 1 | 14278500 | 142845000 | 6 | 161025000 | 161040000 | 11 | 51570000 | 51600000 | 24 | 9180000 | 9195000 |
| 1 | 14286000 | 142875000 | 7 | 61785000 | 61800000 | 16 | 33945000 | 33975000 | 24 | 9930000 | 10050000 |
| 1 | 14290500 | 142965000 | 7 | 61965000 | 61980000 | 16 | 46380000 | 46440000 | 24 | 10080000 | 10095000 |
| 1 | 14323500 | 143295000 | 8 | 43080000 | 43110000 | 17 | 22245000 | 22260000 | 24 | 13260000 | 13320000 |
| 1 | 14350500 | 143520000 | 8 | 43785000 | 43800000 | 17 | 45210000 | 45225000 | 24 | 13395000 | 13500000 |
| 2 | 90375000 | 90390000 | 8 | 43815000 | 43830000 | 18 | 105000 | 120000 | 24 | 13635000 | 13710000 |
| 2 | 92265000 | 92325000 | 8 | 86550000 | 86565000 | 18 | 18510000 | 18525000 | 24 | 13800000 | 13875000 |
| 2 | 13300500 | 133050000 | 8 | 86730000 | 86745000 | 19 | 8850000 | 8865000 | 24 | 28575000 | 28590000 |
| 2 | 16213500 | 162150000 | 9 | 66960000 | 66975000 | 19 | 27720000 | 27750000 | 24 | 28785000 | 28800000 |
| 2 | 20934000 | 209355000 | 9 | 68400000 | 68700000 | 20 | 29625000 | 29640000 | 24 | 58815000 | 58875000 |
| 3 | 19662000 | 196635000 | 9 | 68685000 | 68730000 | 21 | 9825000 | 9840000 | 24 | 58965000 | 59040000 |
| | | | | | | 21 | 10710000 | 10725000 | | | |
| | | | | | | 21 | 11055000 | 11070000 | | | |
| | | | | | | 21 | 11115000 | 11160000 | | | |
| | | | | | | 21 | 11175000 | 11190000 | | | |

10. Dispositif selon la revendication 8 ou 9, dans lequel la première séquence de référence est au moins une partie du génome de référence, avec des régions correspondant à une deuxième fenêtre respectant la condition suivante, retirées : la profondeur de séquençage de la deuxième fenêtre n'est pas inférieure à 4 fois la moyenne arithmétique des profondeurs de séquençage de toutes les deuxièmes fenêtres, et facultativement, la profondeur de séquençage de la deuxième fenêtre n'est pas inférieure à 6 fois la moyenne arithmétique des profondeurs de séquençage de toutes les deuxièmes fenêtres ;
la deuxième fenêtre est acquise par glissement d'une fenêtre de taille L2 sur le génome de référence, et facultativement, la taille de pas du glissement est L2 ; et
la profondeur de séquençage de la deuxième fenêtre est le rapport du nombre de lectures se mettant en correspondance avec la deuxième fenêtre à la taille de la deuxième fenêtre ; ou
dans lequel la première séquence de référence est au moins une partie du génome de référence avec les régions concordant avec les deuxièmes fenêtres dans le génome de référence traitées comme suit : attribution de la profondeur de séquençage de la deuxième fenêtre au 98e centile aux profondeurs de séquençage des deuxièmes fenêtres au-delà du 98e centile, facultativement, attribution de la profondeur de séquençage de la deuxième fenêtre au 99e centile aux profondeurs de séquençage des deuxièmes fenêtres au-delà du 99e centile ;
la deuxième fenêtre est acquise par glissement d'une fenêtre de taille L2 sur le génome de référence, et facultativement, la taille de pas du glissement est L2 ; et
la profondeur de séquençage de la deuxième fenêtre est le rapport du nombre de lectures se mettant en correspondance avec la deuxième fenêtre à la taille L2 de la deuxième fenêtre.

11. Dispositif selon l'une quelconque des revendications 8 à 10, comprenant en outre un module de filtrage, qui est configuré pour au moins l'un des points (i) à (iii) ci-dessous :
(i) le fait de retirer du résultat de séquençage des lectures avec des longueurs ne dépassant pas une longueur prédéfinie ;
(ii) le fait de retirer du résultat d'alignement les lectures non mises en correspondance avec une localisation unique dans la première séquence de référence ; et
(iii) le fait de retirer du résultat d'alignement les lectures avec des taux d'erreur pas inférieurs à un taux d'erreur prédéfini, dans lequel le taux d'erreur d'une lecture est le rapport des bases d'au moins l'une parmi des insertions, des délétions et des discordances dans la lecture après alignement.

12. Dispositif selon l'une quelconque des revendications 8 à 11, dans lequel le module de quantification est configuré pour :
(a) le glissement d'une fenêtre de taille L3 sur la première séquence de référence pour obtenir une pluralité de troisièmes fenêtres ;
(b) la détermination des profondeurs de séquençage des troisièmes fenêtres en fonction du résultat d'alignement, dans lequel la profondeur de séquençage de la troisième fenêtre est le rapport du nombre de lectures se mettant en correspondance avec la troisième fenêtre à la taille L3 des troisièmes fenêtres ; et
(c) la détermination de la quantité de lectures mises en correspondance avec le premier chromosome en fonction de la profondeur de séquençage des troisièmes fenêtres contenues dans le premier chromosome ;
dans lequel facultativement le point (b) comprend en outre :
la normalisation de la profondeur de séquençage de la troisième fenêtre, et le fait de prendre la profondeur de séquençage normalisée de la troisième fenêtre en guise de profondeur de séquençage de la troisième fenêtre ;
dans lequel facultativement en outre le point (b) comprend en outre :
la correction de la profondeur de séquençage de la troisième fenêtre en fonction d'une teneur en GC de la troisième fenêtre, et le fait de prendre la profondeur de séquençage corrigée de la troisième fenêtre en tant que profondeur de séquençage de la troisième fenêtre ; dans lequel facultativement la correction est mise en œuvre en utilisant la relation entre la teneur en GC de la troisième fenêtre et la profondeur de séquençage de la troisième fenêtre ; et facultativement, la relation entre la teneur en GC de la troisième fenêtre et la profondeur de séquençage de la troisième fenêtre est établie par régression localement pondérée.

13. Dispositif selon la revendication 12, dans lequel le point (c) comprend :
la détermination d'un coefficient de pondération de lectures se mettant en correspondance avec la troisième fenêtre en fonction de la profondeur de séquençage de la troisième fenêtre ; et
la détermination de la quantité de lectures mises en correspondance avec le premier chromosome en fonction du coefficient de pondération.

14. Dispositif selon l'une quelconque des revendications 8 à 13, dans lequel l'échantillon soumis à un test est un échantillon de sang provenant d'une femme enceinte ;
facultativement, dans lequel le premier chromosome est au moins l'un des chromosomes 13, 18 et 21 d'un fœtus.

15. Produit programme d'ordinateur comprenant une instruction, dans lequel, lorsque le programme est exécuté sur le dispositif selon l'une quelconque des revendications 8 à 14 l'instruction amène l'ordinateur à exécuter le procédé selon l'une quelconque des revendications 1 à 7.
